# EUROPEAN PATENT SPECIFICATION

(11) **EP 1 044 022 B1**
(45) Date of publication and mention of the grant of the patent: **15.03.2006**
(21) Application number: 98957757.2
(22) Date of filing: 10.11.1998
(51) Int. Cl.: A61K 47/48, A61P 31/04, A61P 31/12, A61P 35/00

(54) **COMPOSITIONS AND METHODS FOR TARGETED DELIVERY OF FACTORS**
ZUSAMMENSETZUNGEN UND METHODEN ZUR ZIELGERICHTETEN ABGABE VON BIOLOGISCH AKTIVENFAKTOREN
COMPOSITIONS ET PROCEDES D'ADMINISTRATION CIBLEE DE FACTEURS

(30) Priority: 10.11.1997 US 966940; 24.02.1998 US 75811 P; 26.05.1998 US 86696 P; 06.11.1998 US 107455 P
(43) Date of publication of application: 18.10.2000
(73) Proprietor: CYTIMMUNE SCIENCES, INC., Rockville, MD 20850 (US)
(72) Inventor: TAMARKIN, Lawrence, Rockville, MD 20854 (US); PACIOTTI, Giulio, F., Baltimore, MD 21075 (US)
(74) Representative: Viering, Jentschura & Partner
(86) International application number: PCT/US1998/023931
(87) International publication number: WO 1999/024077

(56) References cited:
- EP-A- 0 667 398
- WO-A-94/21288
- WO-A-96/04313
- MORRIS R. E., ET AL.: "Validation of the biotinyl ligand-avidin-gold technique" J. HISTOCHEM. CYTOCHEM., vol. 40, no. 5, 1992, pages 711-721, XP002108306
- PETERS D. K., ET AL.: "Binding and internalization of biotinylated interleukin-2 in human lymphocytes" BLOOD, vol. 76, no. 1, July 1990, pages 97-104, XP002108307
- HOPKINS C. R., ET AL.: "Early events following the binding of epidermal growth factor to surface receptors on ovarian granulosa cells" EUROPEAN JOURNAL OF CELL BIOLOGY, vol. 24, no. 2, June 1981, pages 259-265, XP002108308
- KANG Y.-H., ET AL.: "Ultrastructural and immunocytochemical study of the uptake and distribution of bacterial lipopolysaccharide in human monocytes" JOURNAL OF LEUKOCYTE BIOLOGY, vol. 48, no. 4, 1990, pages 316-332, XP002108309

## Description

### Technical Field

The present invention relates to a cell delivery system ffor targeted delivery of biologically-active factors, such as cytokines, growth factors, chemotherapeutic agents, nucleic acids, and therapeutic agents.

### Background of the Invention

The introduction of therapeutic agents into specific target cells has been a challenge to scientists for a long time. The challenge of specific targeting of therapeutic agents has long been an issue of treatment of organisms. The challenge is to get an adequate amount of a therapeutic agent to the target cells of an organism without providing too much exposure of the rest of the organism to the therapeutic agent. Such a challenge is seen in drug delivery. Formulations of therapeutic agents have taken advantage of chemical differences in active agents, such as hydrophobicity or hydrophilicity to crudely target active agents. Additionally, size considerations, such as capability to cross the blood-brain barrier, have limited the targeting of therapeutic agents.

One method that has been used with limited success is the targeting of cells that bear a specific receptor and providing an antibody to that receptor that acts as a carrier for a therapeutic agent. The therapeutic agent could be a pharmaceutical agent that is cytotoxic or the therapeutic agent could be a radioactive moiety that causes cell death. The problems inherent in this techniques are the isolation of the specific receptor, the production of an antibody having selective activity for that receptor and no cross-reactivities with other similar epitopes, and the radioactive labeling or attachment of the therapeutic agent to the antibody. A problem attendant to such limited therapeutic delivery is that the therapeutic agent may never be released internally in the targeted cell, the therapeutic agent is not releasably bound to the antibody and therefore, may not be fully active or capable of any activity once it is delivered to the site.

Another example of limited-success target delivery is in the specific targeting of selected genetic sequences into cells. Many techniques for inserting genes into cells have been tried. Such techniques include precipitation techniques, viral techniques, direct insertion with micropipettes and gene "guns", and most crudely, exposure of nucleic acid to cells. A widely used precipitation technique involves calcium phosphate and is used as a coprecipitate with DNA to form insoluble particles. The goal is for at least some of these particles to become internalized within the host cells by generalized cellular endocytosis. This results in the expression of the new or exogenous genes. This technique has a low efficiency for getting exogenous genes into cells with the resulting translation of the genes. The internalization of the genes is non-specific with respect to which cells are transfected because all exposed cells are capable of internalizing the exogenous genes because there is no reliance upon any particular recognition site for the endocytosis. This technique is used widely *in vitro,* but because of the lack of specificity of target cell selection and poor uptake by highly differentiated cells, its use *in vivo* is not contemplated. In addition, its use *in vivo* is limited by the insoluble nature of the precipitated nucleic acids.

Another similar technique involves the use of DEAE-Dextran for transfecting cells *in vitro.* DEAE-Dextran is deleterious to cells and results in non-specific insertion of nucleic acids into cells. This method would not be advisable in *in vivo.*

Other techniques for transfecting cells, or providing for the entry of exogenous genes into cells are also limited. Using viruses as vectors has some applicability for *in vitro* and *in vivo* introduction of exogenous genes into cells. There is always the risk that the presence of viral proteins will produce unwanted effects in an *in vivo* use. Additionally, viral vectors may be limited as to the size of exogenous genetic material that can be ferried into the cells.

Exogenous gene delivery has also been used with liposome entrapped nucleic acids. Liposomes are membrane-enclosed sacs that can be filled with a variety of materials, including nucleic acids. Liposome delivery does not provide for uniform delivery to cells because of uneven filling of the liposomes. Furthermore, liposomes cannot be targeted to specific cellular types. Liposomes suffer from breakage problems, and thus leakage of the nucleic acids at unwanted sites through out the *in vivo* or *in vitro* systems.

Brute force techniques for inserting exogenous nucleic acids include puncturing cellular membranes with micropipettes or gene guns to insert exogenous DNA into a cell. These techniques work well for some procedures, but is not widely applicable. They are highly labor intensive and require very skilled manipulation of the recipient cell. These are not techniques that are simple procedures that work well *in vivo*. There is no specificity for which particular cells receive the nucleic acid beyond the selection of the cell or cells in the target zone. Electroporation, using electrical methods to change the cellular membrane, has been successful *in vitro* for insertion of genes into cells. Again, this technique cannot be used for *in vivo* applications.

There have been some attempts at target delivery of DNA for specific cells that relied upon the presence of receptors for glycoproteins. The delivery system used polycations, such as polylysine, that were noncovalently bonded to DNA, and that were also covalently bonded to a ligand. Such use of covalently bonding of the polycations to a ligand does not allow for the disassembly of the delivery system once the cellular internalization mechanisms begin. This large complexed delivery system, that is covalently bonded together, is very unlike the way nucleic acids are naturally found within cells.

Morris, R.E. et al. (*J. Hisrochem. Cytochem.* (1992) **40**, 711-721) report on the binding, internalization, and intracellular routing of biotinylated ligands in mouse fibroblasts, wherein the trafficking patterns are monitored in the cells by separately applied streptavidin-gold colloids.

The publication of Peters, D.K. and Norback, D.H. (*Blood* (1990) **76**, 97-104) relates to tracking the internalization of interleukin-2 (IL-2) in activated human lymphocytes using biotinylated recombinant IL-2 adsorbed to agarose-straptavidin, which is used to label the cells. For subsequent detection streptavidin-bound gold is added to the cells.

Hopkins, C.R. and Boothroyd, B. (*Eur. J. Cell Biol.* (1981) **24**, 259-265) also disclose the application of two separate compositions, namely a colloidal gold/avidin complex in conjunction with biotinylated epidermal growth factor (EGF), in order to localize EGF receptors on the surface of ovarian granulosa cells.

Kang, Y.H. et al. (*J. Leukocyte Biol.* (1990) **48,** 316-332) report on the general mechanisms of bacterial lipopolysaccharide (LPS) uptake and distribution in human monocytes using a LPS-specific primary antibody and an anti-mouse IgG linked to colloidal gold.

European Patent Application EP 0 667 398 is directed to a method and an apparatus for detecting a target DNA sequence without the requirement for an operation of labeling the DNAs with chemical substances.

International Patent Application WO 94/21288 relates to compositions for reducing the toxicity of normally toxic biologically active factors by admixing them with a colloidal metal.

Finally, International Patent Application WO 96/04313 discloses poly-specific immunoconjugates that are useful for diagnosis and therapy of diseases caused by multi-drug resistant cells.

Targeted delivery of specific factors to specific cells woultd be important in selective activation or control of the immune system. Currently, there are only crude techniques for immune suppression or activation. The immune system is a complex interactive system of the body that involves a wide variety of components, including cells, cellular factors which interact with stimuli from both inside the body and outside the body. Aside from its direct action, the immune system's response is also influenced by other systems of the body including the nervous, respiratory, circulatory and digestive systems.

One of the better known aspects of the immune system is its ability to respond to foreign antigens presented by invading organisms, cellular changes within the body, or from vaccination. Some of the first kinds of cells that respond to such activation of the immune system are phagocytes and natural killer cells. Phagocytes include among other cells, monocytes, macrophages, and polymorphonuclear neutrophils. These cells generally bind to the foreign antigen, internalize it and may destroy it. They also produce soluble molecules that mediate other immune responses, such as inflammatory responses. Natural killer cells can recognize and destroy certain virally-infected embryonic and tumor cells. Other factors of the immune response include both complement pathways which are capable of responding independently to foreign antigens or acting in concert with cells or antibodies.

One of the aspects of the immune system that is important for vaccination is the specific response of the immune system to a particular pathogen or foreign antigen. Part of the response includes the establishment of "memory" for that foreign antigen. Upon a secondary exposure, the memory function allows for a quicker and generally greater response to the foreign antigen. Lymphocytes in concert with other cells and factors, play a major role in both the memory function and the response.

Vaccines have long been used to stimulate the immune response to protect the organism. Aluminum compounds have been used to form water-insoluble antigenic substances for vaccination purposes. Metals have also been used in capsular polysaccharide metal complex vaccines. Such uses have been limited to the use of the complexes for prophylaxis and treatment of bacterial diseases. Selected metals have also been used as components of stable adjuvant emulsion compositions. It is known in the art that aluminum, as the monostearate, or in the form of hydrated salts of fatty acids, are emulsifying agents, or stabilizers of the emulsion in the vaccine composition.

Targeted delivery of specific therapeutic agents or biologically active factors for the treatment of diseases or pathologies are not currently available. Existing therapies for the treatment of diseases and pathological conditions, including but not limited to, genetic diseases, congenital diseases and acquired diseases such as bacterial infections, viral infections, cancer, immune deficiency diseases, autoimmune diseases, psychiatric diseases, cardiovascular diseases, reproductive dysfunction, somatic growth dysfunction, stress related diseases, muscular dystrophy, osteoporosis, ocular diseases, allergies, and transplantation rejection, require administration of doses of biologically-active factors that have widespread effects throughout the body. These therapies are not specifically targeted to the affected organs for direct delivery of a biologically active factor.

For example, current treatments for cancer include administration of chemotherapeutic agents and other biologically active factors such as cytokines and immune factors. The administration of chemotherapeutic agents to the entire body creates toxic and adverse side effects such as organ damage, loss of senses such as taste and feel, and hair loss. Many chemotherapeutic agents are designed to kill rapidly dividing cells which indiscriminately effect the hematopoetic system and the gastrointestinal system leading to changes in blood and immune cells, vomiting, gastric distress and weight loss. Administration of immune factors, such a cytokines, to the entire body system leads to activation of unwanted immune responses and inhibition of other immune functions. Such therapies provide treatment for the condition, but come with a wide array of side effects that must then be treated. In addition, bolus administration of a drug may not be optimal because of rapid clearance.

Other types of treatment of biological conditions, including diseases, could use nucleic acids. Examples of such therapeutic treatments include gene replacement, antisense gene therapy, triplex gene therapy and ribozyme-based therapy. However, to be successful, an effective means for the delivery of the therapeutic agent to specific cell types and locations, and across cellular, nuclear and other membranes, is required.

Alteration of gene activity can be accomplished many ways. For example, oligonucleotides that are complementary to certain gene messages or viral sequences, such as antisense compounds, have been shown to have an inhibitory effect, for example, against viruses. By creating an antisense nucleic acid composition that hybridizes with the targeted RNA message of cells or viruses the translation of the message into protein can be interrupted or prevented. In this fashion gene activity can be modulated.

The ability to deactivate specific genes provides great therapeutic benefits. In tissue culture, antisense oligonucleotides have inhibited infections by herpes-viruses, influenza viruses and the human immunodeficiency virus that causes AIDS. It may also be possible to target antisense oligonucleotides against mutated oncogenes. Antisense technology also holds the potential for regulating growth and development. However, in order for the gene therapy to work, antisense therapeutic compounds must be delivered to the targeted site.

Another type of gene therapy modified gene activity using sense nucleic acids. Defective genes are replaced or supplemented by the administration of nucleic acids that are not subject to the defect. For example, the administered normal nucleic acids could be a DNA molecule that inserts into a chromosome, or may be present in extracellular DNA. and produces functional RNA, which in turn leads to the desired gene product. In this fashion gene defects and deficiencies in the production of the gene product may be corrected.

Still further gene therapy has the potential to augment the normal genetic complement of a cell. For example, the gene therapy technique known as intracellular immunization allows for the intracellular presence of desired gene products. The desired gene product is then expressed on the surface of the cell, perceived by the body as foreign, and the cells expressing the gene product are eliminated by the body's own immune system. However, this approach currently is not feasible due to a lack of effective gene delivery systems that facilitate the taargeted delivery of such a therapeutic agent to a specific site.

Gene therapy may also be used as a method of delivering drugs *in vivo.* For example, if genes that code for therapeutic compounds can be delivered to endothelial cells, the gene products would have facilitated access to the blood stream. Currently, one method for gene delivery to cells is to remove cells from the body, expose the cells to the nucliec acids *ex vivo* and then reintroduce the cells into the body. Alternatively, gene therapy has been accomplished *in vivo* by the injection of naked DNA, DNA-containing liposomes, and the injection of viral or bacterial DNA-containing vectors. Retroviral vectors can be used to deliver genes *ex vivo* to isolated cells, which are then infused back into the patient. However, retroviral vectors have some drawbacks, such as being able to deliver genes only to dividing cells, random integration of the gene to be delivered, potentially causing unwanted genetic alterations, and possibly reverting back to an infectious wild-type retroviral form.

Triplex DNA technology is another form of gene therapy that utilizes oligonucleotides and compounds that specifically bind to particular regions of duplex DNA, thereby inactivating the targeted gene. An advantage of triplex DNA technology is that only a single copy of the oligonucleotide or compound is required to alter gene expression because the binding is at the DNA level, not the mRNA level. A drawback of triplex DNA technology, however, is that the oligonucleotide or compound must pass through not only the cellular membrane, but also the microbial membrane in the case of treating microbial infections, or the nuclear membrane in the case of altering eukaryotic gene function or expression of foreign DNA integrated into chromosomal DNA.

Another gene therapy technology relates to the therapeutic use of ribozymes for the treatment of genetic disorders. Ribozymes are catalytic RNA molecules that consist of a hybridizing region and an enzymatic region. Ribozymes may in the future be engineered so as to specifically bind to a targeted region of nucleic acid sequence and cut or otherwise enzymatically modify the sequence so as to alter its expression or translation into gene product.

Various biologically-active factors have been isolated from humans or animals which have been reported to have therapeutic efficacy. These compounds include, but are not limited to, such powerful biologically-active molecules as cytokines and growth factors. However, it has been found that when these various factors are isolated and purified from natural sources or genetically engineered material, and then injected into a human or animal, they often cause severe side effects and exhibit unwanted toxicity. Because of this toxicity, it has been difficult to use the compounds therapeutically. In addition, it has been difficult to use the active compounds as antigens to produce antibodies against the molecules. Additionally, the toxicity of such molecules, when used in treatment of disease, is related to its higher concentration or presence in sites in which the factor is not generally found. If such powerful factors could be specifically targeted to the site where the factor is needed, without exposure to other sites where side effects would occur, then the problems associated with its general administration could be overcome.

There is a great need for compositions and methods for targeted delivery systems. These delivery systems could be used for targeted delivery to specific cells or organs of genetic material such as genes, polynucleotides, and antisense oligonucleotides that can be used in gene therapy. More specifically, there is a need for compositions that can facilitate the transport of genetic compounds and other drugs and therapeutic compounds across cellular membranes. What is also needed are delivery systems for delivery to specific cell types and organs of therapeutic agents and biological factors to effect the cells or the surrounding environment.

What is needed are compositions and methods for target specific delivery of therapeutic agents to only the target cells. It would be preferable for some administrations and treatnients if the therapeutic agent is internalized by the targeted cells. Once inside the cell, the therapeutic agent should be sufficiently released from the transport system such that the therapeutic agent is active. For example, if the therapeutic agent is exogenous nucleic acids comprising a gene, it should be transcribed if necessary, translated and expressed. Such compositions and methods should be able to deliver therapeutic agents to the target cells efficiently.

There is also a need for a therapeutically effective composition with reduced toxicity, that may be used in therapies for a wide range of immune diseases, cancers, viral diseases and bacterial diseases. In addition, there is a need for a composition that can reduce the toxicity of biologically-active compositions that are toxic if found in too great a concentration or in the wrong location.

### Summary of the Invention

The present invention relates to a cell delivery system as defined in claim 1 for targeted delivery of therapeutic agents into specific cells. The therapeutic agents are taken up by specific cells because of specific receptors on the cells, and are preferably internalized within the cells by receptor-mediated endocytosis. Thus, in a mixture of different cell types, the therapeutic agents are internalized only by cells having the selected receptor and cells lacking the receptor are unaffected.

The cell delivery system of the present invention provides a novel and versatile approach to targeted cell delivery systems. A preferred embodiment of the present invention comprises a delivery structure or platform, to which compositions to be delivered, or cell-specific targeting molecules, are attached. For example, in one embodiment, one member of a binding group is bound to the delivery platform, and the complementary member of the binding group is bound to a therapeutic agent or effector molecule, or is bound to a selected cell specific ligand or targeting molecule. In a more preferred embodiment, one of the complementary members of the binding group is bound to a cell-specific ligand, the targeting molecule, and another of the complementary members of the binding group is bound to a therapeutic agent or effector molecule.

The delivery system of the present invention provides novel treatments comprising targeted combinatorial therapeutics. The present invention comprises compositions and methods for the targeted drug delivery to specific cells. The delivery structure or platform provides a surface for the binding, preferably reversible, though applications may comprise irreversible binding, of elements to be administered to the organism or cells. In one preferred embodiment, the element bound is a member of a binding group. The binding group members may be selected from all such known paired binding groups including but not limited to antibody/antigen; enzyme/substrate; and streptavidin/biotin. The binding group members may then be bound to a cell-specific ligand or a therapeutic agent. The cell-specific ligand or targeting molecule, comprises any cell specific marker, including those specific to one or a small number cellular types or markers that are widely expressed throughout a cellular type, organ or system. The therapeutic agents comprise agents such as biologically active agents, pharmaceutical agents, radioactive or cell deleterious agents, nucleic acids, or other compounds capable of effecting cellular activity.

One embodiment of such a composition comprises a delivery structure or platform with a member of a binding group reversibly bound to it. A preferred embodiment of the present invention comprises colloidal gold as a platform that is capable of binding a member of a binding group to which targeting molecules and effector molecules are bound to create a targeted gene delivery system that preferably employs receptor mediated endocytosis of cells to provide internal delivery of the therapeutic agent. In a more preferred embodiment, the binding group is streptavidin/biotin and the targeting molecule is a cytokine and the effector molecule is a therapeutic agent. Embodiments of the present invention may also comprise binding the effector molecules or targeting molecules in a less specific method such as by using polycations.

In connection with the cell delivery system the present invention also involves the preparation of the targeted delivery system and administration and delivery of the targeted delivery system to the selected cells. It is contemplated in the present invention that the therapeutic agents of the compositions will be active or effective at the cell site. Such activity can be in any form known to those skilled in the art and includes cellular death, production of functioning proteins, production of cellular products, enzymatic activity, export of cellular products, production of cellular membrane components or nuclear components. The delivery to the targeted cells may be achieved by such methods as those used for *in vitro* techniques such as addition to cellular cultures or media.

Another preferred embodiment of the present invention comprises a cell delivery system in which a biologically active factor or therapeutic agent is associated directly with the delivery platform, for example, the binding of a cytokine composition to colloidal gold. Such compositions can be used to reduce the toxicity associated with the administration of therapeutic agents or can be used in vaccine formulations. A composition of the present invention comprises an admixture of a colloidal metal, such as gold chloride (HAuCl₄) in combination with a substance which normally is toxic to a human or animal or a substance capable of producing an immune response, wherein the composition when administered to a human or animal is less or non-toxic. Such methods and compositions may be used in the treatment of bacterial infections, viral infections, cancer and immune disease therapies, including autoimmune diseases, such as rheumatoid arthritis and acquired immune deficiency. One advantage of the present invention is that smaller amounts of a biologically-active factor can be administered than in previously known systems because the factor is targeted directly to a site, or the factor is less toxic for general administration because of the association with the delivery platform. The platform-bound, biologically-active factor may also serve as an immobilized source of releasable biologically-active factor - a constant release depot.

The targeted delivery of the present invention may be used for the simultaneous and/or sequential targeted stimulation of specific components of the immune system. Components of the immune system include such as B cells. T cells, antigen-presenting cells, phagocytes, macrophages, and other immune cells. Because several cellular types of the immune system may all have the same cell marker, a "component-specific targeting molecule" may target several cell or tissue types,

The invention may also be used for increasing the efficacy with which antigens and vaccines induce an immune response i.e. for the stimulation of a specific individual component of the immune system with a composition by the simultaneous presentation of an antigen with an immune component-specific targeting molecule.

The present invention may be used for the simultaneous stimulation of many different individual immune components through the presentation of specific component-stimulating compositions. One embodiment of such a composition comprises a delivery platform with an antigen in combination, with a component-specific immunostimutating agent. The present invention may also be used for the sequential stimulation of the immune cells by providing component-stimulating compositions at one or more steps in an immune response cascade of interacting factors and cells.

Additionally, it is contemplated in the present invention that the cell delivery system described herein can be used for stimulation of an immune response or the suppression of an immune response. Administration of component-specific immunostimulating agents for the suppression of immune responses can be used to control autoimmune diseases or organ rejection.

Therefore, it is an object of the present invention to provide a versatile cell delivery system for effecting cells with therapeutic agents.

It is another object of the present invention to provide a cell delivery system for targeted delivery of therapeutic agents *in vitro.*

Yet another object of the present invention is to provide a cell delivery system for the targeted delivery of therapeutic agents to cells having a specific receptor.

A further object of the present invention is to provide a cell delivery system for delivery of therapeutic agents into cells by receptor-mediated endocytosis.

Still another object of the present invention is to provide a targeted cell delivery system that is capable of binding and delivering a selected therapeutic agent using a specific targeting molecule.

It is an object of the present invention to provide a cell delivery system that are capable of reducing the toxic effects of biologically-active factors.

A further object of the present invention is to provide a cell delivery system useful for the slow release of biologically-active factors.

A still further object of the present invention to provide a reliable and facile cell delivery system useful for enhancing an immune response.

It is another object of the present invention to provide a cell delivery system useful for improving vaccine efficacy.

Yet another object of the present invention is to provide a cell delivery system useful for the targeted stimulation of individual immune components in a specific manner.

Another object of the present invention is to provide a cell delivery system comprising component-specific immunostimulating agents that are capable of effecting a particular component of the immune system.

Still another object of the present invention is to provide a cell delivery system useful for suppressing the immune responses.

These and other objects, features and advantages of the present invention will become apparent after a review of the following detailed description of the preferred embodiment.

### Brief Description of The Drawings

This patent contains at least one color photograph. Copies of this patent with the color photographs will be provided by the Patent and Trademark Office upon request and payment of the necessary fee.
Figure 1 is a schematic drawing of a preferred embodiment of the present invention.
Figure 2 is a graph showing the saturable binding kinetics of the delivery platform with TNF-α.
Figure 3 illustrates the effect of colloidal gold on the generation of a murine-anti-murine IL-6 antibody response.

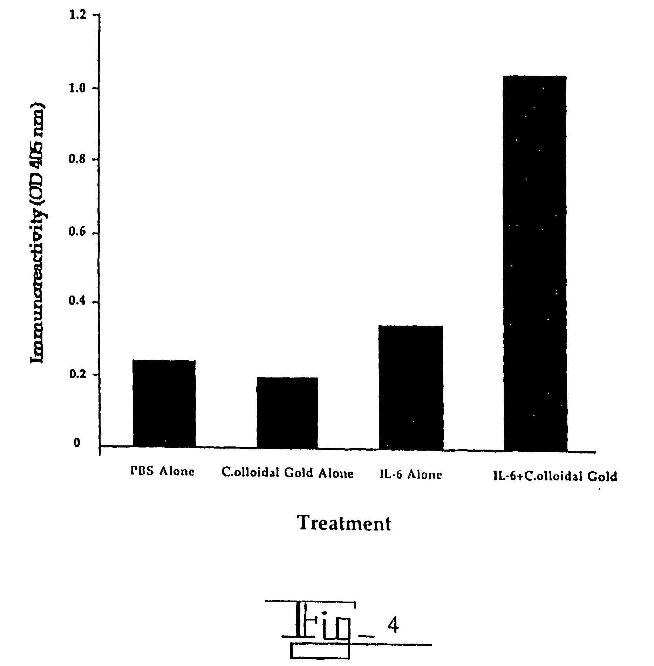

Figure 4 illustrates the increase in immunoreactivity of murine-anti-murine IL-6 antibody response in cells activated with IL-6 bound to colloidal gold over those activated with IL-6 or colloidal gold alone.

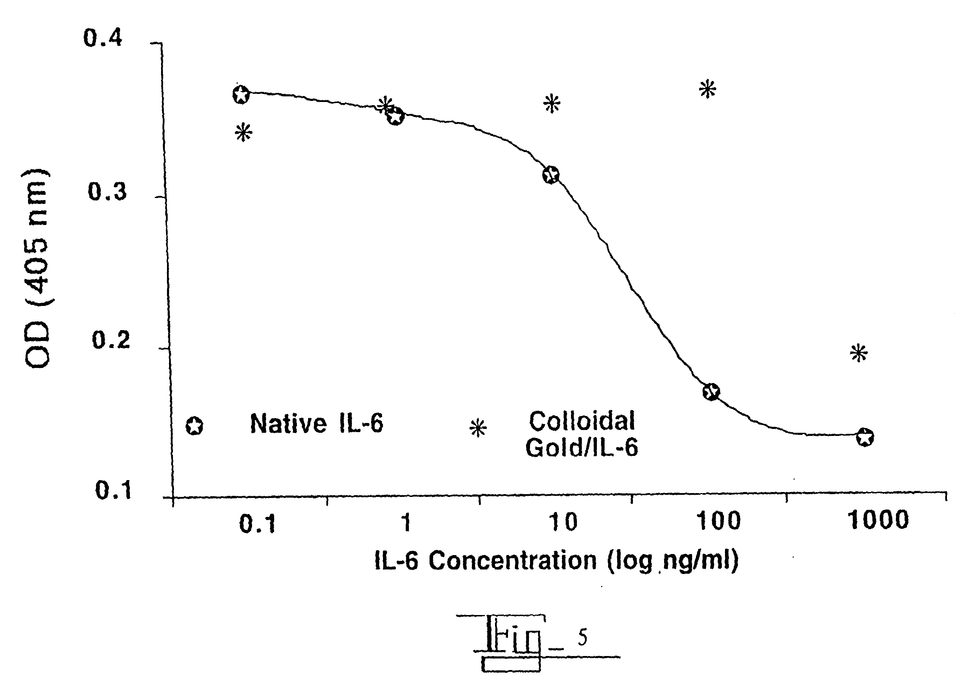

Figure 5 illustrates the efficiency with which gold binds IL-6.
Figure 6 illustrates the retention of biologic activity of IL-I after treatment with gold.
Figure 7 illustrates the effect of time on the release of IL-2 from colloidal gold.
Figure 8 illustrates the effect of dilution on the release of TNFα from colloidal gold.

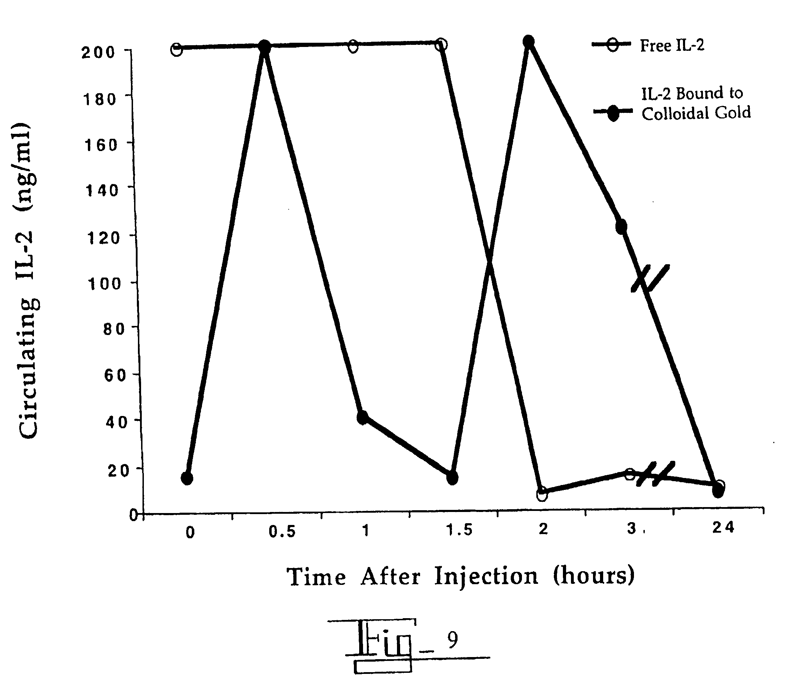

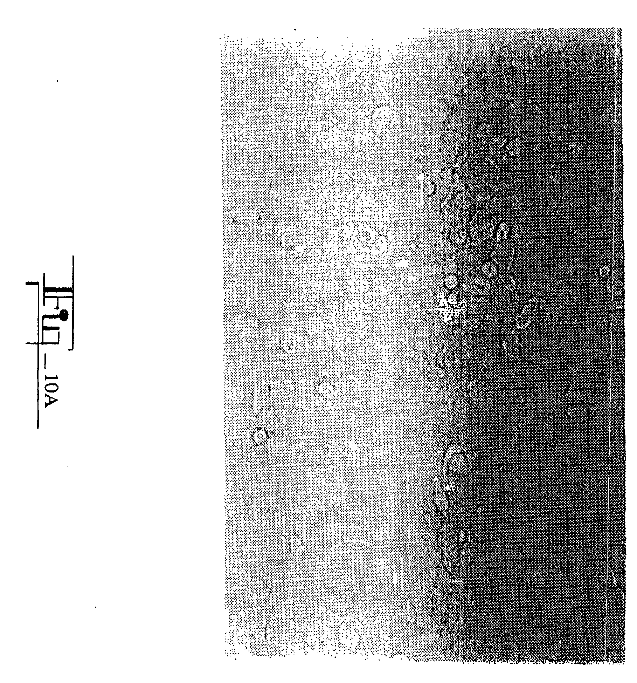

Figure 9 illustrates the in-vivo release of IL-2 bound to colloidal gold.
Figures 10a-d illustrate the internalization of colloidal gold-bound IL-1β by MCF-7 cells.
Figure 11 illustrates the effect on immunoreactivity of colloidal gold which is coupled with TNFα, IL-6 and IL-1β.
Figure 12 illustrates the in vitro internalization of EGF/CG/IL-1β complex by macrophages.
Figure 13 illustrates the in vitro internalization of EGF/CG/TNF-α complex by dendritic cells.
Figure 14 illustrates the in vitro internalization of EGF/CG/IL-6 complex by B-cells.
Figure 15 illustrates the in vitro internalization of EGF/CG/IL-2 complex by T-cells.
Figure 16 illustrates the in vitro internalization of EGG/Histone/DNA/Colloidal Gold chimera by the human breast cancer cells, MCF-7.
Figure 17 illustrates the control transfection using the EGF/Histone/DNA/Colloidal gold chimera and the human breast cancer cells. HS-578-T.

### Detailed Description

The present invention relates to a cell delivery system as defined in claim 1 for targeted delivery of therapeutic agents into cells. More particularly the present invention comprises compositions of a delivery structure or platform with another element bound to it. A preferred embodiment of the present invention comprises a colloidal metal as a platform that is capable of binding a member of a binding group to which targeting molecules and effector molecules are bound to create a targeted gene delivery system. Such a system preferably uses receptor-mediated endocytosis of cells to achieve internal delivery of a therapeutic agent. In a most preferred embodiment the bindings group is streptavidin/biotin and the targeting molecule is a cytokine and the effector molecule is a therapeutic agent. Embodiments of the present invention may also comprise binding the effector molecules or targeting molecules in a less specific method, without the use of binding partners, such as by using polycations or proteins. Other embodiments of the present invention comprise binding or association of an effector molecule or a targeting molecule directly to the delivery structure or platform.

The present invention comprises a cell delivery system for targeted delivery of therapeutic agents that use colloidal metals as a platform. Such colloidal metals may bind, reversibly or irreversibly, molecules that interact with either therapeutic agents or targeting molecules. The integrating molecules may either be specific binding molecules, such as members of a binding pair, or may be rather nonspecific integrating molecules that bind less specifically. An example of such less specific binding is the binding of nucleic acids by polycationic molecules such as polylysine or histones. The present invention contemplates the use of integrating molecules such as polycationic elements known to those skilled in the art including polylysine, protamine sulfate, histones or asialoglycoproteins.

The members of the binding pair comprise any such binding pairs known to those skilled in the art, including antibody-antigen pairs, enzyme-substrate pairs; receptor-ligand pairs; and streptavidin-biotin. Novel binding partners may be specifically designed. An essential characteristic of the binding partners is the specific binding between one of the binding pair with the other member of the binding pair such that the binding partners are capable of being joined specifically. Another desired characteristic of the binding members is that one member of the pair is also capable of binding or being bound to either an effector molecule or a targeting molecule, and the other member is bound to the delivery platform.

Though not wishing to be limited by any particular theory, it is theorized that changes in pH that occur in endosome formation in receptor-mediated endocytosis can be utilized for internal cellular delivery of therapeutic agents. When colloidal metal compositions are at neutral pH, the composition is a sol. When the pH is lowered, the colloidal metal composition precipitates and aggregates. A lowered pH includes a range of pH values of approximately less than 7 to approximately 2. A preferred lowered pH is that which is found in an endosome, which is approximately pH 4. to approximately pH 6, most preferably pH 5.6. The aggregation of the metal particles in the endosome effectively reduces the surface area of the metal particle for binding, which results in the release of bound materials from the metal carrier.

The delivery platform is preferably a colloidal metal composition. For example, in a preferred embodiment, the delivery platform is a colloidal gold particle that has a negative charge at an approximately neutral pH. This negative charge prevents the attraction and attachment of other negatively charged molecules. In contrast, positively charged molecules are attracted to and bind to the colloidal gold particle. Such positively charged molecules comprise polycations including polylysine, histones, protamine sulfate, and asialoglycoproteins. The histones contemplated in the present invention may be a mixture of different histones, one specific type of histone, or combinations of specific histones. The positively charged molecules may also comprise members of a binding pair, such as antibody-antigen or streptavidin/biotin. After the binding of the positively charged molecules to the delivery platform, the complementary binding member or the effector molecules can be bound.

The effector molecules comprise both molecules for cell or target selection known herein as targeting molecules, and molecules that provide an effect once delivered to the target, the therapeutic agents or biologically active factors. The association of the effector molecules with the metallic platform may either be through specific means, using the members of the binding pairs and their specific binding, or through less specific means. For example, less specific means includes ionic interactions.

Use of the specific binding characteristic of the binding members comprises the binding of one of the members of the binding pair to the therapeutic agent and the complementary binding member to the metallic platform. The attachment of one binding member to a therapeutic agent is accomplished through means that are well known in the art, and is exemplified in the binding of biotinylated therapeutic agents. The biotin may be bound through such methods as protein binding or may be incorporated into nucleic acids through synthesis using biotinylated nucleosides. The binding of biotin to therapeutic agents may be accomplished using chemical binding such as providing specific linkers or addition of active groups to the therapeutic agent or targeting molecules.

It is the use of such binding members that provides for one aspect of the flexibility of the present invention. Any desired therapeutic agent can be bound to one of the members of the binding pair. The complementary binding member is then associated with the metallic platform. Thus, any therapeutic agent can be delivered using the metallic platform of the present invention. Futhermore, any targeting molecule can be bound to one of the members of the binding group.

To provide specificity for cell or target selection for the delivery of the therapeutic agent, cell-specific targeting molecules are also attached to the metallic platform. Such cell-specific targeting molecules include any molecules that bind to structures on cells receptors found in cellular membranes. Such cell-specific targeting molecules also include receptors or parts of receptors that may bind to molecules found in the cellular membranes or free of cellular membranes. Examples of such cell-specific targeting molecules include Interleukin-1 ("IL-1"), Interleukin-2 ("IL-2"), Interleukin-3 ("IL-3"), Interleukin-4 ("IL-4"), Interleukin-5 ("IL-5"), Interleukin-6 ("IL-6"), Interleukin-7 ("IL-7"), Interieukin-8 ("IL-8"), Interleulcin-10 ("IL-10"), Interleukin-11 ("IL- 11"), Interleukin-12 ("IL-12"), Interleukin-13 ("IL-13"), Interleukin-15 ("IL-15"), Interleukin-16 ("IL-16"). Interleukin-17 ("IL-17"), Interleukin-18 ("IL-18"), lipid A, phospholipase A2, endatoxins, staphylococcal enterotoxin B and other toxins. Type I Interferon. Type II Interferon, Tumor Necrosis Factor ("TNFα"). Transforming Growth Factor-β ("TGF-β"), Lymphotoxin. Migration Inhibition Factor, Granulocyte-Macrophage Colony-Stimulating Factor ("CSF"). Monocyte-Macrophage CSF, Granulocyte CSF, vascular epithelial growth factor ("VEGF"), Angiogenin, transforming growth factor ("TGFα"), heat shock proteins, carbohydrate moieties of blood groups, Rh factors, fibroblast growth factor and other inflammatory and immune regulatory proteins, hormones, such as growth hormone, insulin, glucogon, parathyroid hormone, luetinizing hormone, follicle stimulating hormone, and luetinizing hormone releasing hormone, cell surface receptors, antibodies, nucleic acids, nucleotides. DNA, RNA, sense nucleic acids, antisense nucleic acids, cancer cell specific antigens; such as MART, MAGE, BAGE, and HSPs; mutant p53; tyrosinase; antoimmune antigens: receptor proteins, glucose, glycogen, phospholipids, and monoclonal and/or polyclonal antibodies, and basic fibroblast growth factor.

The elements that bind to the delivery device or platform may be bound by any method. Such elements comprise the integrating molecules, specific targeting molecules, therapeutic agents or biologically active factors. The following example illustrates a binding method for the integrating molecules but the present method is not limited to only this element, and any elements contemplated in the present invention can be bound in this method. The integrating molecules, either the less specific binding molecules such as the polycationic components or the specific binding molecules, the members of the binding pair, may be bound to the colloidal metal by any method. One preferred method is to reconstitute the integrating molecules, in water at approximately a pH of 1 to 3 pH units above the integrating molecules' isoelectric point, pI. Approximately 100 to 1,000 µg, preferably 150 to 800 µg, and most preferably 200 to 500 µg of the integrating molecules are then incubated with the colloidal metal. The duration of the incubation is not critical, but is preferably from approximately 2 to 48 hours, more preferably 18 to 36 hours.

Following incubation, the colloidal metal platform, bound with the integrating molecule, is optionally stabilized by incubating it overnight with 1% by volume of a 1-100% solution of polyethylene glycol (PEG). Alternatively, cysteine, phospholipids, Brij 58, or sulfhydryl-containing compounds can be used to stabilize the colloidal metal platform. The solution is then centrifuged, followed by optional stabilization of the resulting pellet by incubation with cysteine, phospholipids, sulfhydryl-containing compounds, or a 1% solution of human serum albumin (HSA) in protein reconstitution buffer. Typically, between 90 and 95% of the component is bound by this method, as determined by immunoassay measurement of the unbound biologically-active factor in the supernatant.

Binding of an element in this manner changes the physical properties of the colloidal metal. Prior to binding, the colloidal metal cannot be filtered through a 0.22 micron filter. After binding, the colloidal complex is easily filtered. This difference suggests that the metal is no longer present as a colloid, but as an ionic solution.

The amount of colloidal metal that is used in the present invention is between approximately 0.001 mg/ml and 1.0 mg/ml with the more preferred amount of colloidal metal being between approximately 0.01 mg/ml and 0.1 mg/ml. The amount of the composition according to the present invention to be administered to in *vivo* or *in vitro* varies according to the desired application, the molecules to be delivered, the cells targeted for delivery and the mode of administration.

Other methods of preparations of colloidal compositions and uses are e.g. found in the related US Patent 6,274,552.

The effector molecules are then bound to the metallic platform through the integrating molecules. The effector molecules may either be cell-specific targeting molecules or the therapeutic agent. The effector molecules may be bound directly to the integrating molecules, such as histones, or may bind through specific interaction of the binding members. If the methods comprise specific interaction using binding members, one of the binding members functions as the integrating molecule bound to the platform and the complementary binding member is bound to the effector molecule. For example, one embodiment of the present invention comprises the binding members of streptavidin and biotin, and the streptavidin functions as the integrating molecule and is associated with the colloidal metal platform. The biotin is bound to the effector molecules. In a further preferred embodiment, the biotin is bound to the cell-specific targeting molecule, for example, TNF-α. The biotin is also bound to the therapeutic agent, such as truncated forms of toxins which are devoid of extracellular binding domains.

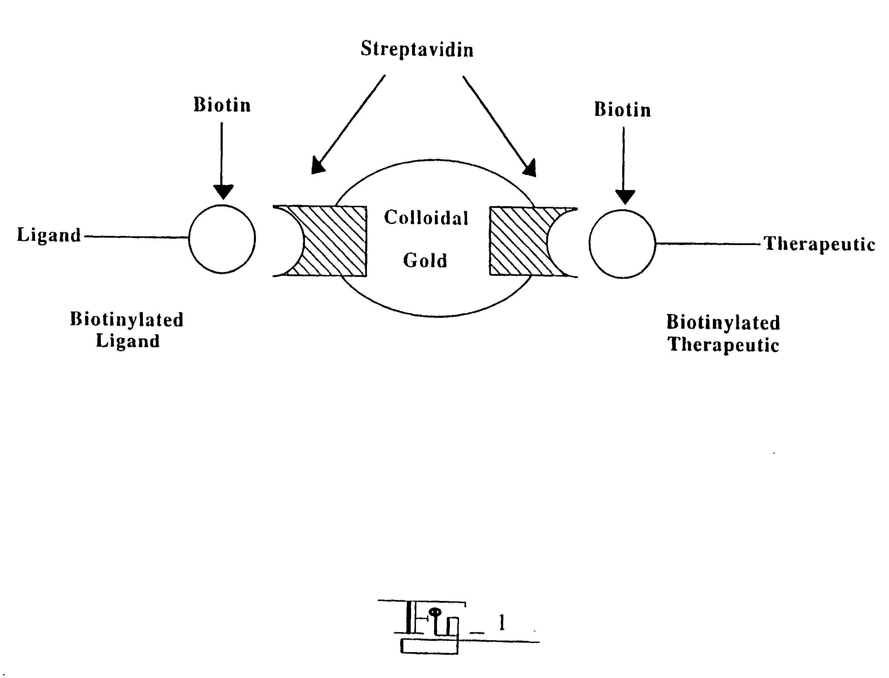

An embodiment of the current invention comprises the use of streptavidin bound to colloidal gold as a platform for developing and customizing targeted combinatorial therapeutics. The present invention involves the targeting of therapeutic agents using ligand/receptor binding and the process of receptor mediated endocytosis (RME). The embodiment uses streptavidin bound to colloidal gold to co-localize biotinylated ligands and therapeutics. In essence, by using streptavidin bound to colloidal gold, the problems of binding two chemically distinct moieties onto a single colloidal gold particle are eliminated. Rather the chemistries of the target and therapeutic are now based on the well-characterized binding of biotin to streptavidin. A diagrammatic representation of this embodiment is depicted in Fig. 1.

### Several Embodiments of the Present Invention

The cell delivery system of the present invention comprises the variations and combinations of mixtures of the elements disclosed herein and of their binding capabilities and methods of activity. For example, an embodiment of the present invention comprises the cell-specific targeting molecules bound directly to the metallic platform and the therapeutic agent being bound to the metallic platform through either specific or less specific binding by integrating molecules. An embodiment of the present invention comprises the therapeutic agent bound directly to the colloidal metal platform and the cell-specific targeting molecules bound through either specific or less specific binding by integrating molecules. Another embodiment of the present invention comprises the binding of both the cell-specific targeting molecules and the therapeutic agent to the metallic platform through specific or less specific binding by integrating molecules or the direct binding of the cell-specific targeting molecules and the therapeutic agent to the metallic platform. A still further embodiment of the present invention comprises the cell-specific targeting molecules bound to the metallic platform through binding by the binding members and the therapeutic agent bound to the metallic platform through less specific binding means. A contrasting embodiment of the present invention comprises binding the cell-specific targeting molecules bound to the metallic platform through binding by the less specific integrating molecule binding and the therapeutic agent bound to the metallic platform by the binding of complementary binding members. Other combinations and variations of such embodiments are contemplated.as part of the present invention.

The elements that are bound to the delivery platform, including targeting molecules, integrating molecules, and effector molecules, such as therapeutic agents and biologically-active factors, may be bound to the platform in any combination. For example, two effector molecules may be bound to the platform such that a targeting and effector complex can be produced by binding a cytokine and a cytokine receptor to a colloidal metal particle. Alternatively, a gene delivery system can be produced by binding a nucleic acid component and a cytokine receptor to a colloidal metal particle. A therapeutic system can be produced by binding a chemotherapeutic agent and a cytokine receptor to the colloidal metal particle. Additionally, an antibody to a cancer antigen can be the targeting molecule and can be bound to the colloidal metal along with a chemotherapeutic agent. In delivering the chemotherapeutic agent to a target cell, such as a cancer cell, low concentrations of the chemotherapeutic agent can be used thereby reducing the systemic toxicity of the chemotherapeutic agent.

Another embodiment of the present invention is to use the binding of two or more biologically-active factors to the same colloidal metal particle as a method for binding one molecule to a cell surface receptor, while the second molecule is released into the extracellular space proximal to the target cell. Such a slow release depot serves to deliver biologically active molecules to their specific site of action.

Yet another embodiment of the present invention is to use the binding of two or more elements to the same delivery platform, such as a colloidal metal particle, as a method for using specific molecular transport mechanisms for one of these components to cross biological barriers, which then carries the second component or group along with the first. For example, one of the components bound to the delivery platform can be glucose. Glucose, which has a specific blood-brain transport system, is bound to a colloidal metal, along with an effector molecule such as a biologically active molecule. The active transport of glucose across the blood-brain barrier serves as a conduit for any associated biologically-active factors, which in and of themselves are unable to cross this biological barrier. These compositions then serve as vehicles for the delivery of therapeutic molecules to areas usually unavailable to the delivered factor, such as the brain.

### A Preferred Delivery Platform

Colloidal metals are a preferred delivery platform, though other materials that function in a similar manner are contemplated as being comprised in the present invention. Though not wishing to be bound by any particular theory, the role of colloidal metal, and more preferably, colloidal gold, in the present invention is not merely to act as a docking site for the associated elements. To the contrary, its role may be as important as that of the bound elements. For example, some drugs only become active after being internalized by a cell. Drugs using RME as the method for internalization often remain trapped and are ultimately destroyed in the membrane organelle, the endosome. The inactivation of the drug is often the result of a decrease in the endosomal pH. It is this physiologic change in the pH of the endosome that the value of the colloidal gold is appreciated. We have observed that protein-bound colloidal gold undergoes physical changes when exposed to an acidic, i.e., endasomal-like, environment In a very simple experiment, colloidal gold bound with saturating concentrations of BSA was dialyzed against a MES buffer at two different pH (7.4 and 5.6). The results of this experiment showed that colloidal gold dialyzed against physiologic (i.e., 7.4) pH remained in its colloidal state. However the same preparation dialyzed against the acidic pH formed very large aggregates. By forming such aggregates, the colloidal gold facilitates the intracellular release of a putative drug by lysing the endosome.

### Use of the cell delivery system

The cell delivery system of the invention can be used for the manufacture of a medicament for the treatment of a medical condition selected from the groups consisting of bacterial infections, viral infections, cancer, and autoimmune diseases. Administration of the medicament may include direct application to the target cells or such routes of administration as used by pharmaceuticals, including but not limited to formulations include those suitable for oral, rectal, transdermal, ophthalmic. (including intravitreal or intracameral) nasal, topical (including buccal and sublingual), vaginal or parenteral (including subcutaneous, intramuscular, intravenous, intradermal, intratracheal, and epidural) administration. The formulations may conveniently be presented in unit dosage form and may be prepared by conventional pharmaceutical techniques. Such techniques include the step of bringing into association the active ingredient and the pharmaceutical carrier(s) or excipient(s). In general, the formulations are prepared by uniformly and intimately bringing into association the compositions with liquid carriers or finely divided solid carriers or both, and then, if necessary, shaping the product.

### Definitions

The terms "toxic reaction," and "toxicity," as used herein, include the following cellular responses: fever, edema, including cerebral edema; psychosis; autoimmune diseases; hemorrhage; shock, including hemorrhagic shock; sepsis; cachexia; or death.

The terms "biologically-active factors" and "therapeutic agents" include Interleukin-1 ("IL-1"), Interleukin-2 ("IL-2"), Interleukin-3 ("IL-3"), Interleukin-4 ("IL-4"), Interteukin-5 ("IL-5"), Interleukin-6 ("IL-6"), Interleukin-7 ("IL-7"), Interleukin-8 ("IL-8"), Interleukin-10 ("IL-10"), Interleukin-11 ("IL-11"), Interleukin-12 ("IL-12"), Interleukin-13 ("IL-13"), Interleukin-15 ("IL-15"), Interleukin-16 ("IL-16"), Interleukin-17 ("IL-17"), Interleukin-18 ("IL-18"); lipid A, phospholipase A2, endotoxins, staphylococcal enterotoxin B and other toxins, Type I Interferon, Type II Interferon, Tumor Necrosis Factor ("TNFα"), Transforming Growth Factor-β ("TGF-β"), Lymphotoxin, Migration Inhibition Factor, Granulocyte-Macrophage Colony-Stimulating Factor ("CSF"), Monocyte-Macrophage CSF, Granulocyte CSF, vascular epithelial growth factor ("VEGF"), Angiogenin, transforming growth factor ("TGFα"), heat shock proteins, carbohydrate moieties of blood groups, Rh factors, fibroblast growth Factor, hormones, such as growth hormone, insulin, glucogen, parathyroid hormone, leutinizing hormone, follicle stimulating hormone, and luetinizing hormone releasing hormone cell surface receptors, antibodies, chemotherapeutic agents, and other inflammatory and immune regulatory proteins, nucleic acids, nucleotides, DNA, RNA, sense nucleic acids, antisense nucleic acids, cancer cell specific antigens; such as MART, MAGE, BAGE, and HSPs; and immunotherapy drugs, such as AZT, pharmacueticals and other therapeutic drugs.

Examples of therapeutic agents and organisms to be treated are found in the following table.

**TABLE 1 Organisms and Selected Therapeutic Agents**

| | |
|---|---|
| *Bacteria* | |
| Mycobacterium tuberculosis | Isoniazid, rifampin, ethambutol, pyrazinamide, streptomycin, clofazimine, rifabutin, fluoroquinolones such as ofloxacin and sparfloxacin |
| Mycobacterium avium | Rifabutin, rifampin, azithromycin, clarithromycin, fluoroquinolones |
| Mycobacterium leprae | Dapsone |
| Chlamydia trachomatis | Tetracycline, doxycyline, erythromycin, ciprofloxacin |
| Chlamydia pneumoniae | Doxycycline, erythromycin |
| Listeria monocytogenes | Ampicillin |
| *Fungi* | |
| Candida albicans | Amphotericin B, ketoconazole, fluconazole |
| Cryptococcus neoformans | Amphotericin B, ketoconazole, fluconazole |
| *Protozoa* | |
| Toxoplasma gondii | Pyrimethamine, sulfadiazine, clindamycin, azithromycin, clarithromycin, atovaquone |
| Pneumocystis carinii | Pentamidine, atovaquone |
| Cryptosporidium sp. | Paromomycin, diclazaril |
| *Virus* | |
| Herpes simplex virus type 1 | Acyclovir, trifluorouridine and other and type 2 antiviral nucleoside analogs, foscomat, antisense oligonucleotides, and triplex-specific DNA sequences |
| Cytomegalovirus | Foscarnet, ganciclovir |
| HIV | AZT. DDI. DDC, foscarnat, viral protease inhibitors, peptides, antisense oligonucleotides, triplex and other nucleic acid sequences |
| Influenza virus type A and B | Ribavirin |
| Respiratory syncytial virus | Ribavirin |
| Varizella zoster virus | Acyclovir |

The term "cell-specific targeting molecule" includes Interleukin-1 ("IL-1"), Interleukin-2 ("IL-2"), Interleukin-3 ("IL-3"), Interleukin-4 ("IL-4"), Interleukin-5 ("IL-5"), Interleukin-6 ("IL-6"), Interleukin-7 ("IL-7"), Interleukin-8 ("IL-8"), Interleukin-10 ("IL-10"), Interleukin-11 ("IL-11"), Interleukin-12 ("IL-12"), Interteukin-13 ("IL-13"), Interleukin-15 ("IL-15"), Interleukin-16 ("IL-16"), Interleukin-17 ("IL-17"), Interleukin-18 ("IL-18"), lipid A, phospholipase A2, endotoxins, staphylococcal enterotoxin B and other toxins. Type I Interferon, Type II Interferon, Tumor Necrosis Factor ("TNFα"), Transforming Growth Factor-β("TGF-β"), Lymphotoxin, Migration Inhibition Factor. Granulocyte-Macrophage Colony-Stimulating Factor ("CSF"), Monocyte-Macrophage CSF, Granulocyte CSF, vascular epithelial growth factor ("VEGF"), Angiogenin, transforming growth factor ("TGFα"), heat shock proteins, carbohydrate moieties of blood groups. Rh factors, fibroblast growth factor and other inflammatory and immune regulatory proteins, hormones, such as growth hormone, insulin, glucogon, parathyroid hormone, luetinizing hormone, follicle stimulating hormone, and luetinizing hormone releasing hormone, cell surface receptors, antibodies, nucleic acids, nucleotides, DNA, RNA. sense nucleic acids, antisense nucleic acids, cancer cell specific antigens: such as MART, MAGE, BAGE, and HSPs; mutant p53; tyrosinase; antoimmune antigens: receptor proteins, glucose, glycogen, phospholipids, and monoclonal and/or polyclonal antibodies, and basic fibroblast growth factor.

The term "colloidal metal", as used herein, includes any water-insoluble metal particle or metallic compound dispersed in liquid water (a hydrosol). The colloidal metal may be selected from the metals in groups IA, IB, IIB and IIIB of the periodic table, as well as the transition metals, especially those of group VIII. Preferred metals include gold, silver, aluminum, ruthenium, zinc, iron, nickel and calcium. Other suitable metals may also include the following in all of their various oxidation states: lithium,sodium, magnesium, potassium, scandium, titanium, vanadium, chromium, manganese, cobalt, copper, gallium, strontium, niobium, molybdenum, palladium, indium, tin, tungsten, rhenium, platinum, and gadolinium. The metals are preferably provided in ionic form, (preferably derived from an appropriate metal compound) for example the Al³⁺, Ru³⁺, Zn²⁺, Fe³⁺, Ni²⁺ and Ca²⁺ ions. A preferred metal is gold, particularly in the form of Au³+. An especially preferred form of colloidal gold is HAuCl₄ (E-Y Laboratories, Inc., San Mateo, California). Another preferred metal is silver, particularly in a sodium borate buffer, having the concentration of between approximately 0.1 % and 0.001%, and most preferably, approximately a 0.01% solution. Preferably, the color of such a colloidal silver solution is yellow and the colloidal particles range from 1 to 40 nanometers. Such metal ions may be present in the complex alone or with other inorganic ions.

### Some Embodiments of the Present Invention cell delivery system for Gene Therapy

The present invention comprises a cell delivery system for targeted delivery of exogenous nucleic acids or genetic information into specific cells. The exogenous genetic information is taken up by specific cells because of specific receptors on the cells, and is preferably, internalized within the cells by receptor-mediated endocytosis. Thus, in a mixture of different cell types, the exogenous nucleic acids are internalized only by cells having the selected receptor and cells lacking the receptor are unaffected.

The present invention comprises a cell delivery system for the transfection of specific cells. One embodiment of such a cell delivery system comprises nucleic acid bound to polycations that are bound to colloidal metals. A preferred embodiment of the present invention comprises colloidal gold as a platform that is capable of binding targeting molecules and effector molecules to create a targeted gene delivery system that employs receptor-mediated endocytosis of cells to achieve transfection. In a more preferred embodiment, the targeting molecule is a cytokine and the effector molecule is exogenous genetic material such as DNA or RNA. This embodiment may also comprise integrating molecules such as polycations.

In connection with the cell delivery system the present invention also involves the preparation of the targeted gene delivery system and delivery of the targeted gene delivery system to the cells for transfection. It is contemplated in the present invention that the nucleic acids of the cell delivery system will be eventually translated and expressed by the cell. Such expression can be in any form known to those skilled in the art and includes functioning proteins, production of cellular products, enzymatic activity, export of cellular products, production of cellular membrane components, or nuclear components. The methods of delivery to the targeted cells may be such methods as those used for *in vitro* techniques such as addition to cellular cultures.

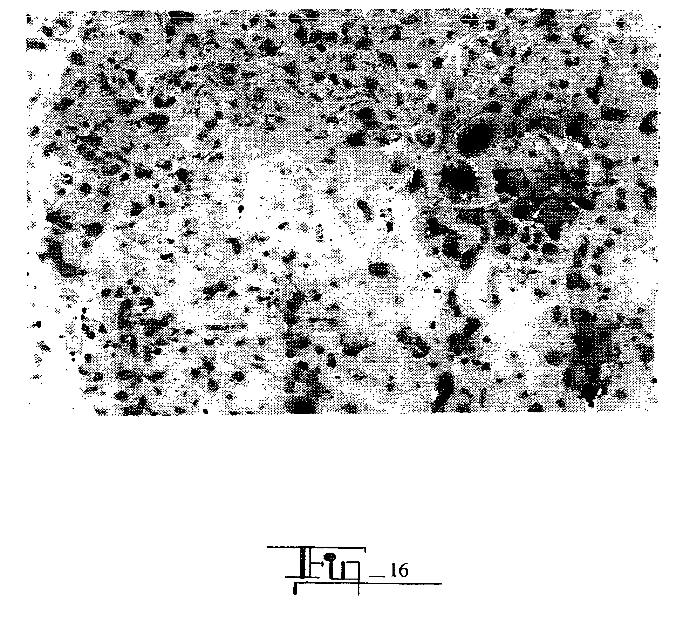

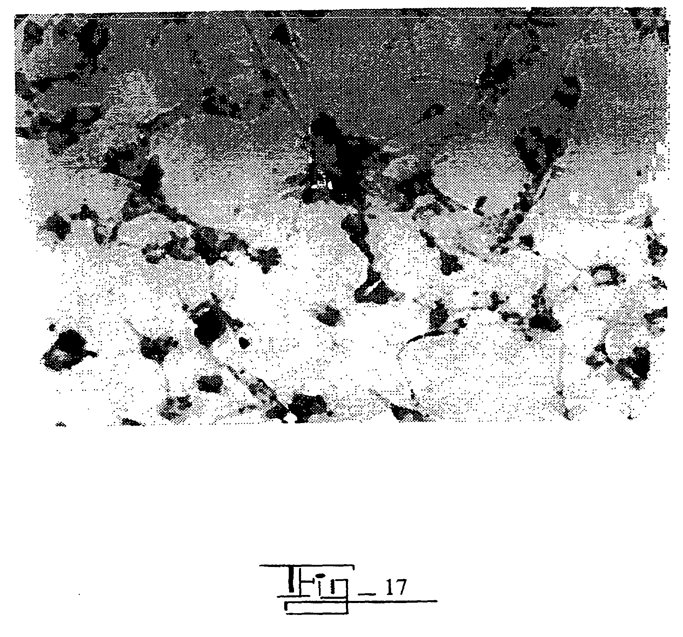

Figures 16 and 17 show experiments using embodiments of the present invention. Figure 16 demonstrates successful receptor targeted gene delivery in the human breast cancer cells, MCF-7. An EGF/histone chimera was constructed on 40 nm colloidal gold. Upon precipitation and resuspension plasmid DNA coding for the pSV beta-galactosidase gene was incubated and re-centrifuged as described above. The presence of the enzyme (beta-galactosidase) was detected by incubating the cells with the substrate X-gal. Blue-green stain indicates cells the presence of the gene product whereas the pink stain indicates the presence of the EGF/Histone/DNA/Colloidal Gold Chimera.

Figure 17 shows a control transfection using the EGF/Histone/DNA/Colloidal gold chimera. Human breast cancer cells, HS-578-T, were treated at the same time as MCF-7 cells with the identical chimera described for Figure 16. The cells were then treated with X-gal to detect the presence of transfected gene. Figure 17 clearly shows that HS-578-T cells although capable of binding the chimera, as seen by the black staining, were not able to be internalized. Consequently, no blue-green color is seen, indicating a lack of transfection.

### Immune Stimulation

The present invention relates to a cell delivery system useful for enhancing an immune response and increasing vaccine efficacy through the simultaneous or sequential targeting of specific immune components. More particularly, specific immune components including, but not limited to, antigen presenting cells (APCs), such as macrophages and dendritic cells, and lymphocytes, such as B cells and T cells, are individually effected by one or more component-specific immunostimulating agents. An especially preferred embodiment of the invention provides for activation of the immune response using a specific antigen in combination with the component-specific immunostimulating agents. As used herein, component-specific immunostimulating agent means an agent, that is specific for a component of the immune system, and that is capable of effecting that component, so that the component has an activity in the immune response. The agent may be capable of effecting several different components of the immune system, and this capability may be employed in the methods and compositions of the present invention. The agent may be naturally occurring or can be generated and manipulated through molecular biological techniques or protein receptor manipulation.

The activation of the component in the immune response may result in a stimulation or suppression of other components of the immune response, leading to an overall stimulation or suppression of the immune response. For ease of expression, stimulation of immune components is described herein, but it is understood that all responses of immune components are contemplated by the term stimulation, including stimulation, suppression, rejection and feedback activities.

The immune component that is effected may have multiple activities, leading to both suppression and stimulation or initiation or suppression of feedback mechanisms. The present invention is not to be limited by the examples of immunological responses detailed herein, but contemplates component-specific effects in all aspects of the immune system.

The activation of each of the components of the immune system may be simultaneous, sequential, or any combination thereof. In one embodiment of a method of the present invention, multiple component-specific immunostimulating agents are administered simultaneously. In this method, the immune system is simultaneously stimulated with four separate preparations, each containing a composition comprising a component-specific immunostimulating agent. Preferably, the composition comprises the component-specific immunostimulating agent associated with colloidal metal. More preferably, the composition comprises the component-specific immunostimulating agent associated with colloidal metal of one sized particle or of different sized particles and an antigen. Most preferably, the composition comprises the component-specific immunostimulating agent associated with colloidal metal of one sized particle and antigen or of differently sized particles and antigen.

The inventors have found that they could use certain component-specific immunostimulating agents provide a specific stimulatory, up regulation, effect on individual immune components. For example, Interleukin-1β (IL-1β) specifically stimulates macrophages, while TNF-α (Tumor Necrosis Factor alpha) and Flt-3 ligand specifically stimulate dendritic cells. Heat killed Mycobacterium butyricum and Interleukin-6 (IL-6) are specific stimulators of B cells, and Interleukin-2 (IL-2) is a specific stimulator of T cells. Compositions comprising such component-specific immunostimulating agents provide for specific activation of macrophages, dendritic cells. B cells and T cells, respectively. For example, macrophages are activated when a composition comprising the component-specific immunostimulating agent IL-1β is administered. A preferred composition is IL-1β in association with colloidal metal, and a most preferred composition is IL-1β in association with colloidal metal and an antigen to provide a specific macrophage response to that antigen.

Many elements of the immune response are necessary for an effective vaccination. An embodiment of a method of simultaneous stimulation is to administer four separate preparations of compositions of component-specific immunostimulating agents comprising 1) IL- 1β for macrophages, 2) TNF-α and Flr-3 ligand for dendritic cells, 3) IL-6 for B cells, and 4) IL-2 for T cells. The component-specific immunostimulating agent compositions may be administered by any routes known to those skilled in the art, and may use the same route or different routes, depending on the immune response desired.

In another embodiment of the present invention, the individual immune components are activated sequentially. For example, this sequential activation can be divided into two phases, the primer phase and the immunization phase. The primer phase comprises stimulating APCs, preferably macrophages and dendritic cells, white the immunization phase comprises stimulating lymphocytes, preferably B cells and T cells. Within each of the two phases, activation of the individual immune components may be simultaneous or sequential. For sequential activation, a preferred method of activation is activation of macrophages followed by dendritic cells, followed by B cells, followed by T cells. A most preferred method is a combined sequential activation wherein there is simultaneous activation of the macrophages and dendritic cells, followed by the simultaneous activation of B cells and T cells. This is an example of methods and compositions of multiple component-specific immunostimulating agents to initiate several pathways of the immune system.

The cell delivery system of the present invention can also be used to enhance the effectiveness of any type of vaccine. The present methods enhance vaccine effectiveness by targeting specific immune components for activation. Compositions comprising component-specific immunostimutating agents in association with colloidal metal and antigen are used for increasing the contact between antigen and specific immune component. Examples of diseases for which vaccines are currently available include cholera, diphtheria, Haemophilus, hepatitis A, hepatitis B, influenza, measles, meningitis, mumps, pertussis, small pox, pneumocococcal, pneumonia, polio, rabies, rubella, tetanus, tuberculosis, typhoid, Varicella-zoster, whooping cough, and yellow fever.

The combination of route of administration and the packaging used to deliver the antigen to the immune system is a powerful tool in designing the desired immune response. Various packaging methods, such as liposomes, microcapsules, or microspheres, that can provide long-term release of immune stimulating compositions can be used in connection of the present invention. These packaging systems act like internal depots for holding antigen and slowly releasing antigen for immune system activation. For example, a liposome may be filled with a composition comprising an antigen and component-specific immunostimulating agents associated with colloidal metal. Additional combinations are colloidal gold particles studded with viral particles which are the active vaccine candidate or are packaged to contain DNA for a putative vaccine. The gold particle would also contain a cytokine which could then be used to target the virus to specific immune cells. Furthermore, one could create a fusion protein vaccine which targets two or more potential vaccine candidates and generate a vaccine for two or more applications. The particles may also include immunogens which have been chemically modified by the addition of polyethylene glycol which may release the material slowly.

The antigen/component-specific immunostimulating agent/metal complex is slowly released from the liposome and is recognized by the immune system as foreign and the specific component to which the component-specific immunostimutating agent is directed activates the immune system. The cascade of immune response is activated more quickly by the presence of the component-specific immunostimulating agent and the immune response is generated more quickly and more specifically.

Other cell delivery systems contemplated in the present invention include using antigen/component-specific immunostimulating agent/colloidal metal complexes in which the colloidal metal particles have different sizes. Sequential administration of component-specific immunostimulating agents may be accomplished in a one dose administration by use of these differently sized colloidal metal particles. One dose would include four independent component-specific immunostimulating agents complexed an antigen and each with a differently sized colloidal metal particle. Thus, simultaneous administration would provide sequential activation of the immune components to yield a more effective vaccine and more protection for the population. Other types of such single-dose administration with sequential activation could be provided by combinations of differently sized colloidal metal particles and liposomes, or liposomes filled with differently sized colloidal metal particles.

Use of such systems as described above are very important in providing vaccines that can be administered in one dose. One dose administration is important in treating animal populations such as livestock or wild populations of animals. One dose administration is vital in treatment of populations that are resistant to healthcare such as the poor, homeless, rural residents or persons in developing countries that have inadequate health care. Many persons, in all countries, do not have access to preventive types of health care, as vaccination. The reemergence of infectious diseases, such as tuberculosis, has increased the demand for vaccines that can be given once and still provide long-lasting, effective protection. The cell delivery systems of the present invention provide such effective protection.

The cell delivery system of the present invention can also be used to treat diseases in which an immune response occurs, by stimulating or suppressing components that are a part of the immune response. Examples of such diseases include, Addison's disease, allergies, anaphylaxis. Bruton's syndrome, cancer, including solid and blood borne tumors, eczema, Hashimoto's thyroiditis, polymyositis, dermatomyositis, type 1 diabetes mellitus, acquired immune deficiency syndrome, transplant rejection, such as kidney, heart, pancreas, lung, bone, and liver transplants, Graves' disease, polyendocrine autoimmune disease, hepatitis, microscopic polyarteritis, polyarteritis nodosa, pemphigus, primary biliary cirrhosis, pernicious anemia, coeliac disease, antibody-mediated nephritis, glomerulonephritis, rheumatic diseases, systemic lupus erthematosus, rheumatoid arthritis, seronegative spondylarthritides, rhinitis, sjogren's syndrome, systemic sclerosis, sclerosing cholangitis, Wegener's granulomatosis, dermatitis herpetiformis, psoriasis, vitiligo, multiple sclerosis, encephalomyelitis, Guillain-Barre syndrome, myasthenia gravis, Lambert-Eaton syndrome, sclera, episclera, uveitis, chronic mucocutaneous candidiasis, urticaria, transient hypogammaglobulinemia of infancy, myeloma, X-linked hyper IgM syndrome, Wiskott-Aldrich syndrome, ataxia telanviectasia, autoimmune hemolytic anemia, autoimmune thrombocytopenia, autoimmune neutropenia. Waldenstrom's macroglobulinemia, amyloidosis, chronic lymphocytic leukemia, and non-Hodgkin's lymphoma.

The cell delivery system of the present invention may comprise component-specific immunostimulating agents. A system may comprise one component-specific immunostimulating agent or multiple component-specific immunostimulating agents. Preferred embodiments of the system comprise component-specific immunostimulating agents in association with colloidal metals. More preferred embodiments comprise systems comprising component-specifc immunostimulating agents in association with colloidal metals and other elements for specifically targeting the effect of the component-specific immunostimulating agents, including antigens, receptor molecules, nucleic acids, pharmaceuticals, chemotherapy agents, and carriers. The system of the present invention may be delivered to the immune components in any manner. In one embodiment, an antigen and a component-specific immunostimulating agent are bound to a colloidal metal in such a manner that a single colloidal metal particle is bound to both the antigen and the immunostimulating agent.

### In Summary

In one disclosed embodiment of the present invention, the specific immune components stimulated are macrophages, dendritic cells. B cells, and T cells. In a preferred embodiment, the cell delivery system comprise component-specific immunostimulating agents. In a more preferred embodiment, the compositions comprise component-specific immunostimulating agents in association with colloidal metal. In another disclosed embodiment an antigen and a component-specific immunostimulating agent are bound to a colloidal metal, such as colloidal gold, and the resulting chimeric molecule is present to the immune component.

The present invention also relates to the presentation of antigen and component-specific immunostimulating agents in a variety of different delivery platforms or carrier combinations. For example, a preferred embodiment includes administration of an antigen in association with component-specific immunostimulating agents and colloidal gold in a liposome carrier. Additional combinations are colloidal gold particles studded with viral particles which are the active vaccine candidate or are packaged to contain DNA for a putative vaccine. The gold particle would also contain a cytokine which could then be used to target the virus to specific immune cells. Such embodiments provide for an internal vaccine preparation that slowly releases antigen to the immune system for a prolonged response. This type of vaccine is especially beneficial for one-time administration of vaccines. All types of carriers including liposomes and microcapsules are contemplated in the present invention.

### Toxicity Reduction and Vaccine Administration

The present invention comprises a cell delivery system for the in vitro administration of normally toxic biologically-active factors. Generally, the systems according to the present invention comprise an admixture of a colloidal metal in combination with a substance which normally is toxic whereas the system is less toxic or non-toxic. The compositions optionally include a pharmaceutically-acceptable carrier, such as an aqueous solution, or excipients, buffers, antigen stabilizers, or sterilized carriers. Also, oils, such as paraffin oil, may optionally be included in the composition.

The cell delivery system of the present invention can be used to treat certain diseases with cytokines or growth factors. By admixing the biologically-active factors with the colloidal metal the toxicity of the biologically-active factor is reduced or eliminated thereby allowing the factor to exert its therapeutic effect. The combination of a colloidal metal with such biologically-active factors reduces toxicity while maintaining or increasing the therapeutic results thereby improving the efficacy as higher concentrations of biologically-active factors may be administered, or by allowing the use of combinations of biologically-active factors. The use of colloidal metals in combination with biologically-active factors therefore allows the use of higher concentrations of biologically-active factors or formerly unusable toxic substances .

One embodiment of the present invention is to use a biologically-active factor associated with the colloidal metal as a vaccine preparation. Among the many advantages of such a vaccine is the reduction of toxicity of normally toxic factors. The vaccine against biologically-active factors may be prepared by any method. One preferred method for preparing a vaccine against biologically-active factors is to admix the selected biologically-active factor with the colloidal metal in a salt-free medium, preferably deionized water. The salt-free medium may optionally be buffered with, for example, Tris buffer. In one embodiment of the invention, the colloidal metal solution is diluted 1: :1 with the solution of biologically-active factors.

The medium should preferably not contain sodium ions. A colloidal gold solution has a light pink color, this color should not change when adding the solution containing the biologically-active factors. If the colloidal gold solution turns from pink to purple, this indicates that the gold has precipitated and cannot be reconstituted for effective immunization. The shelf-life of an admixture of colloidal gold and biologically-active factor(s) is approximately 24 hours.

The vaccine may further comprise a pharmaceutically acceptable adjuvant, including Freund's complete adjuvant. Freund's incomplete adjuvant lipopolysaccharide, monophosphoryl lipid A, muramyl dipeptide, liposomes containing lipid A, alum, muramyl tripeptidephosphatidylethanoloamine, keyhole and limpet hemocyanin. A preferred adjuvant is Freund's incomplete adjuvant, which preferably is diluted 1:1 with the mixture of colloidal metal and biologically-active factor.

An amount of biologically-active factor used in the present invention is between approximately 200 µg and 500 µg of protein bound in 25 ml of gold, which is then sequentially concentrated to 200 µl Ideal concentrations to be administered are approximately 6.5 mg of protein/kg body weight. The actual amount will vary depending upon the particular patient and condition to be treated. The amount of bound biologically-active factor released in the body depends upon the amount of protein initially bound to the colloidal metal and the total concentration of protein administered.

The cell delivery system of the invention can be used for the manufacture of a medicament for the treatment of bacterial infection, viral infection, cancer or autoimmune diseases comprising an effective amount of a colloidal metal admixed with a biologically-active factor or factors, wherein the composition is less or non-toxic. The medicament used can be administered as a vaccine against a normally toxic substance or can be a therapeutic agent wherein the toxicity of the normally toxic agent is reduced thereby allowing the administration of higher quantities of the agent over longer periods of time.

The process by which the medicament is administered is not considered critical. The routes that the medicament may be administered include subcutaneous, intramuscular, intraperitoneal, oral, and intravenous routes. A preferred route of administration is intravenous. Another preferred route of administration is intramuscular.

It is known that Interleukin-2 (IL-2) displays significant therapeutic results in the treatment of renal cancer. However, the toxic side effects result in the death of a significant number of the patients. In contrast, if IL-2 is mixed with colloidal gold, little or no toxicity is observed and a strong immune response occurs. The doses previously used for IL-2 therapy have been on the order of 21x10⁶ units of IL-2 per 70 kg person per day (7x10⁶ units of IL-2 per 70 kg person TID). One unit equals approximately 50 picograms. 2 units equals approximately 0.1 nanograms, so 20x10⁶ units equals 1 milligram. In one example the amount of IL-2 that has been given to rabbits is approximately mg per 3 kg rabbit. In effect, the studies of the effects of the administration of biologically-active factors described herein have included doses of more than 20 times higher than that previously given to humans.

In another example where IL-2 (1 mg per 3 kg animal) was administered to 3 rabbits every third day for a two-week period, all the animals appeared to be clinically sick, and two of the animals died from the apparent toxic effects of the IL-2. When the same dose of IL-2 was combined with colloidal gold and then administered to three rabbits for the same two-week period, no toxicity was observed and a significant antibody response resulted in all three animals. A "positive antibody response" as used herein is defined as a three to fourfold increase in specific antibody reactivity, as determined by direct ELISA, comparing the post-immunization bleed with the preimmunization bleed. A direct ELISA is done by binding IL-2 onto a microtiter plate, and determining the quantity of IgG bound to the IL-2 on the plate, by goat anti-rabbit IgG conjugated to alkaline phosphatase. Therefore, it is thought that the biological effects of the IL-2 remain. As the toxicity effects have been minimized, larger concentrations of IL-2 may be administered if necessary where a larger, more effective immune response is required. [-]

The invention is also directed to the use of the cell delivery system for the manufacture of a medicament for treating a disease comprising one or more biologically-active factors bound to a colloidal metal. After administration, the biologically-active factor is released from the colloidal metal. Though not wishing to be bound by any theory, it is thought that the release is not simpiy a function of the circulation time of the medicament used but is controlled by equilibrium kinetics.

When the colloidal metal: biological active factor complex ("complex") was incubated with cells for 25 days, it was found that only 5% of the biologically-active factor was released from the colloidal metal. Thus, circulation time alone does not explain the mechanism through which the biologically-active factor is released from the complex *in vivo*. However, it has been found that the amount of biologically-active factor released is, in part, dependent upon the concentration of the complex. When various dilutions of the complex were analyzed (CytELIZA assay system Cytlmmune Sciences. Inc., College Park. MD), it was found that the more dilute solutions of the complex released a significantly greater amount of biologically-active factor. For example, there was essentially no release of biologically-active factor in a 1:100 dilution of the complex, whereas over 35,000 pg. of biologically-active factor was released in a 1:100,000 dilution of the same sample of complex.

Therefore, the lower the concentration of the complex, the greater the amount of biologically-active factor released. The higher the concentration of the complex, the lower the amount of biologically- active factor released. Thus due to the continuous dilution of the complex after administration of the medicament used. It is possible to achieve the same therapeutic effect by administering a lower dose of biologically-active factor to a patient than can be administered by previously known methods.

It has also been found that the amount of biologically-active factor released from the colloidal metal:biologically active factor complex is dependent upon the amount of biologically-active factor initially bound to the colloidal metal. More biologically-active factor is released from complex having a greater amount of biologically-active factor initially bound. Thus, the skilled artisan is able to control the amount of biologically-active factor delivered by varying the amount, of biologically-active factor initially bound to the colloidal metal.

These combined properties provide a method by which a large amount of biologically-active factor can be bound to a colloidal metal, thereby rendering the biolocrically active factor less toxic than if administered alone. Then, a small amount of the colloidal metal:biologically-active complex can be administered resulting in the slow release of the biologically-active factor from the complex. This method provides an extended, low dose of the biologically active/therapeutic factor for the treatment of diseases such as cancer and immune diseases.

The cell delivery system of the present invention is useful for the treatment of a number of diseases including both solid tumors as well as blood-borne cancers, such as leukemia; or autoimune diseases, such as rheumatoid arthritis.

This invention is further illustrated by the following examples, which are not to be construed in any way as imposing limitations upon the scope thereof. On the contrary, it is to be clearly understood that resort may be had to various other embodiments, modifications, and equivalents thereof which, after reading the description herein, may suggest themselves to those skilled in the art.

### EXAMPLE I

This example demonstrates that colloidal gold neutralizes otherwise toxic substances and allows for an antibody response. When IL-2 (1 mg per 3 kg animal) is administered to three rabbits every third day for a two-week period, all the animals appear to be clinically sick, and two of the animals died from the apparent toxic effects of the IL-2. When the same dose of IL-2 is combined with colloidal gold and then administered to three rabbits for the same two-week period, no toxicity is observed and a significant antibody response results in all three animals. A positive annbpdy response is defined as a three to fourfold increase in specific antibody reactivity, as determined by direct ELISA, comparing the post-immunization bleed with the pre-immunization bleed. A direct ELISA is done by binding IL-2 onto a microtiter plate, and determining the quantity of IgG bound to the IL-2 on the plate, by goat anti-rabbit IgG conjugated to alkaline phosphatase.

### EXAMPLE II

This example further demonstrates that colloidal gold neutralizes otherwise toxic substances and allows for an antibody response. Endotoxin or lipid A (25, 50, and 100 µg per 35 mg mouse) are administered by subcutaneous injection every fourth day over a two-week period. For ten mice, endotoxin is given "neat" and for the remaining ten, the endotoxin is mixed 1:1 with colloidal gold. The injection volume is made up by adding potassium carbonate/sodium citrate buffer, pH 6.5 at a 1:1 dilution. The same protocol is also used where lipid A is the test drug.

The animals are checked at 15, 30, and 60 minutes following each injection, and then hourly and daily. The surviving animals are tested for a specific antibody response to the toxic substance they were injected with, either endotoxin or lipid A. Most of the animals injected with endotoxin or lipid A combined with colloidal gold survived, while those injected with the neat endotoxin or lipid A died during the two-week test period. In addition, those animals that did survive did have an antibody response to the specific toxin as determined by direct ELISA.

### EXAMPLE III

This example illustrates the effect of colloidal gold on cytokine activity in *vivo.* A group of mice are given IL-2 at a dose close to that given to cancer patients undergoing immunotherapy. In previous experiments. 18 µg of IL-2 tablets given to nude mice reduced implant tumor size, but killed the animals within two weeks. *See* Paciotti, G.F., and Tamarkin, L., Interleukin-2 Differentially Effects the Proliferation of a Hormone-Dependent and a Hormone-Independent Human Breast Cancer Cell Line *In Vitro* and *In Vivo, Anti-Cancer Research,* 8: 1233-1240 (1988), which is hereby incorporated by reference.

The efficacy of gold in a murine model system is tested in the following procedure: a group of mice are treated with IL-2 alone, IL-2 mixed with colloidal gold, colloidal gold alone, or saline solutions delivered through an osmotic mini pump. The mice are treated for seven days, after which they are sacrificed and their lymphocytes harvested. The cells are stained for T-cell or B-cell markers using specific murine monoclonal antibodies for flow cytometric analysis. Activated T- and B-cells are determined by assessing T-cell numbers, helper T-cell to suppresser T-cell ratios, activated cellular IL-2 receptor, B-cell numbers, and natural killer cell ("NK") numbers.

The few animals that survived being treated with IL-2 alone showed an increase in the T-cell number and activity (as determined by IL-2 receptors). Virtually all the animals survived IL-2 treatment in combination with colloidal gold, and these animals showed an increase in both B-cell function (as determined by activated B-cells and total IgG, measured by direct ELISA) an increase in T-cell function (as determined by T-cell number, and activity, using IL-2 receptor numbers as an index of activity), and an increase in NK activity.

### EXAMPLE IV

The following biological experiment shows that colloidal gold reduces the toxicity of lipopolysaccharide (LPS). LPS is the lipid/sugar moiety of bacterial cell walls. When injected into an animal, this molecule mimics many of the clinical responses of septic shock. Thus, mice were injected with varying amounts of LPS in the presence or absence of colloidal gold. Specifically Balbic mice were injected with either 100 or 400 µg of LPS (strain W.E. coli 055:B5; 10 mg/ml in water; Difco Labs) with or without colloidal gold. The pH of the 15 nm colloidal gold mixture (E.Y. Labs) was adjusted to approximately 10, while the pH of the LPS was adjusted to 8 with 0.1 N NaOH. Subsequently, appropriate volumes (i.e., 10 µl for the 100 µg dose and 40 µl for the 400 ug dose) was then added to 500 µl of colloidal gold. The mixture was allowed to stand for 30 minutes and subsequently injected (i.p.) into the mice.

Within 12 hours after the injections, all mice exhibited clinical signs of depression and anergasia. Within 24 hours after the injection control mice in the 400 ug dose began to die. By 72 hours all of the control mice in the 400 ug dose died while 75% of the gold treated mice were alive and began showing signs of clinical improvement (i.e., movement). Furthermore, although subjective, the mice in the 100 µg dose which were treated with gold were more active throughout the 36 hours of observation.

### EXAMPLE V

The following experiment describes the use of colloidal gold as a putative adjuvant for generating mouse antibodies against murine IL-6. This experiment was performed with two goals in mind: First, to determine if colloidal gold could be used as an adjuvant in generating an immune response to "self antigens" (i.e., generating an immune response to a mouse protein using a mouse model); Second, since IL-6 is one of the cytokines thought to be involved in cancer cachexia, metastasis and sepsis, then the ability to generate antibodies in an autologous system may prove advantageous in generating a vaccine to the IL-6 and similar endogenous compounds.

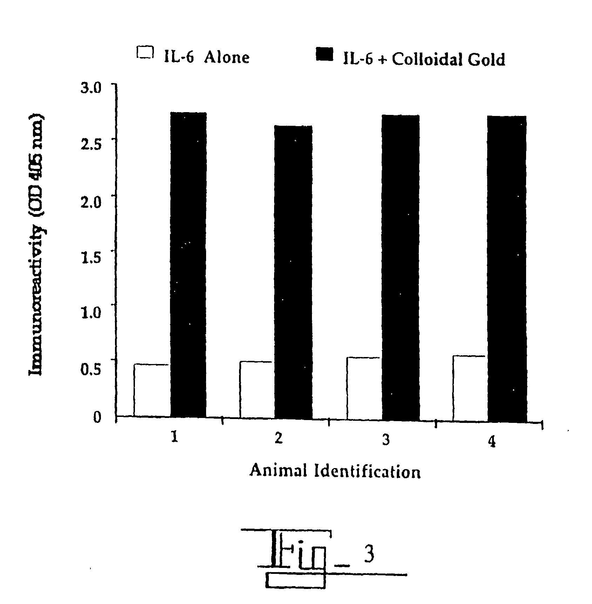

Briefly described, the experiment is as follows. Several mice were immunized with colloidal gold/murine IL-6 mixture as described above. Approximately 3 weeks later, the mice were sacrificed and trunk blood was collected and analyzed for the presence of antibodies to murine IL-6 by a direct ELISA, as described above. The results from the direct ELISA, the determination of the serum antibody titers in mice immunized with murine IL-6 combined with colloidal gold, are illustrated in Figure 3. Figure 3 demonstrates that the mice had generated an antibody response to murine IL-6 thus indicating that the gold is be useful in generating antibodies to endogenous (i.e., selt) toxins as well as cytokines thought to be involved in sepsis, cancer cachexia and metastasis.

Based upon these results, colloidal gold can also be used to generate monoclonal antibodies in transgenic mice through an immune response to "self antigens." Any colloidal gold bound antigen can be used to immunize the transgenic mice, resulting in the *in vivo* generation of Mabs.

### EXAMPLE VI

The following experiment shows that cytokines mixed with colloidal gold retain their biological activity. The model used for these experiments is one which is well known in the art. See Paciotti, G.F., and L. Tamarkin, *Interleukin 1 directly regulates hormone-dependent human breast cancer cell proliferation in vitro,* Mol. Endocrinol., 2: 459-464, 1988; and Paciotti, G.F., and L. Tamarkin, *Interleukin-1 differentially synchronizes estrogen-dependent and estrogen-independent human breast cancer cells in the GoIG I-phase of the cell cycle,* AntiCancer Research, 11: 25-32, 1991. The model is based on the ability of the cytokine. IL-1, to directly inhibit the growth of estrogen-responsive human breast cancer cells. MCF-7. Briefly described, IL-I alone inhibits the growth of these cells through a well-characterized IL-1 receptor on the surface of these breast cancer cells.

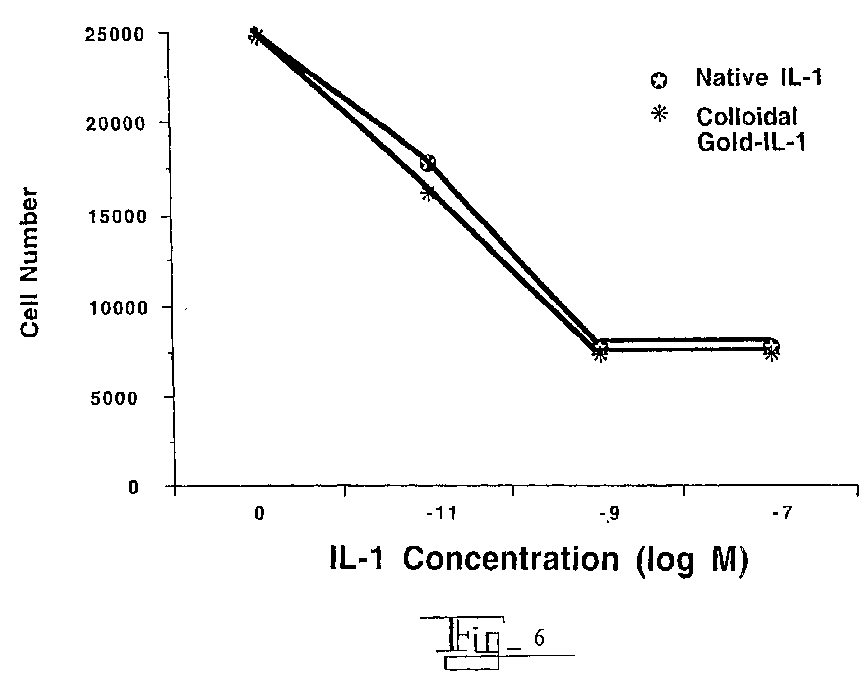

The following experiment shows the ability of IL-1 when mixed with colloidal gold to retain its biological activity by determining its ability to inhibit the growth of these cells. Approximately 8,000 MCF-7 cells were plated in 24-well tissue culture plates. On the next day, 15 nm gold particles were centrifuged at 14,000 rpm for 10 minutes and resuspended in sterile water. Human IL-1α was reconstituted in water to an initial stock of 5 X 10⁻⁵M in water. The pH of the gold and IL-1 was adjusted to approximately 8.0 with 0.1 M NaOH. Prior to mixing, the IL-1 was diluted to a working stock of 2 X 10⁻⁶, 2 X 10⁻⁸ M, and 2 X 10⁻¹⁰ M, which contained 250 µl of the gold (final volume = 0.5 ml). Gold controls consisted of 250 µl of gold and 250µl of sterile water. Subsequently, each working stock was further diluted 1/20 in tissue media resulting in final concentrations of 10⁻⁷, 10⁻⁹, and 10⁻¹¹ M. These solutions along with the appropriate controls were then added directly to the MCF-7 cells. The data presented in Figure 6 are the number of cells present at various days after the addition of IL-1 with or without the gold.

### EXAMPLE VII

The following experiment shows the efficiency with which gold binds cytokine. This experiment demonstrates that the protein is removed from the solution when it is combined with gold and then centrifuged. The experiment used the IL-6 standard from ARI's Cytokit-6. Prior to mixing, the pH of the gold and cytokine solutions were adjusted to pH 9 with 0.1 N NaOH. This protein was either preincubated with gold or water prior to using it in ARI's diagnostic kit for IL-6. Following this incubation, the colloidal gold IL-6 mixture was centrifuged and the supernatants were used to generate a standard curve. As can be seen from Figure 3 the gold was very effective at binding virtually all the IL-6 in the dose-range of the assay, removing the IL-6 from the supernatant. Even at the highest final concentration (1000 ng/ml) of IL-6, the gold removed approximately 90% of the IL-6 in the solutions. This amount is based on the OD of the 1000 ng/ml IL-6/gold supernatant, which is similar to the 100 ng/ml IL-6 standard alone.

### EXAMPLE VIII

The following experiment shows the physical changes in the gold colloid solution upon its mixing with IL-6, a potential antigen for a vaccine. Although the gold particles are approximately 15 nm in size, they cannot be filtered through a 0.22 µm syringe filter. We attribute this to the nature of the gold particles in this colloid mixture. It is theorized that the gold as a colloidal mixture forms aggregates larger than the individual spheres. Although the individual particles are smaller than the pore size of the filter, the aggregates are much larger and thus are not filterable. However, we observed that once the colloidal gold is incubated with protein it easily filters through the 0.22 µm filter. Thus, the binding of a cytokine appears to change the physical interactions of the gold particles with each other; making the gold particles act as single 15 nm particles and enabling the particles to be readily filtered. This experiment defines the nature of the binding of an antigen to the colloidal metal.

### EXAMPLE IX

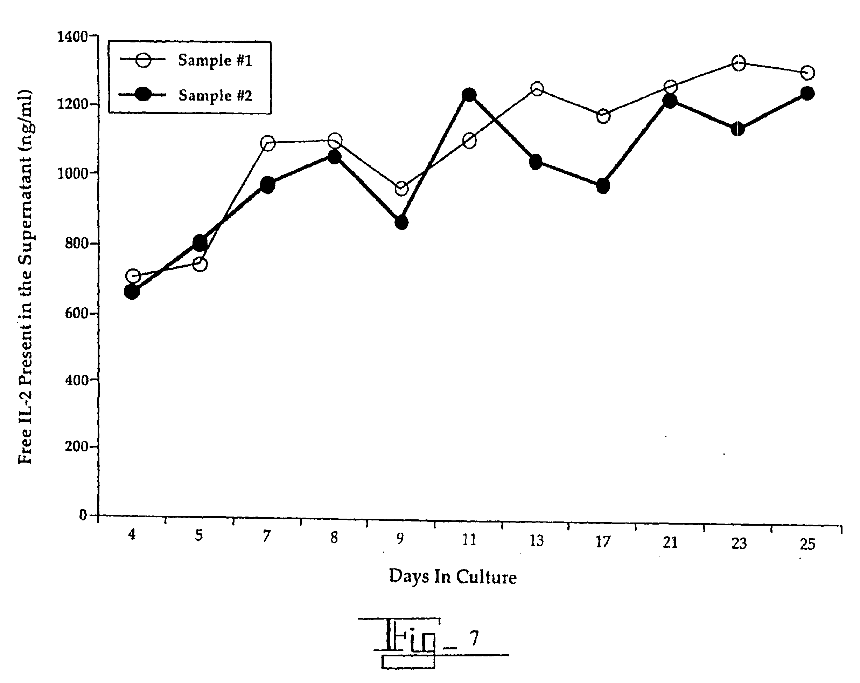

Human IL-2 was reconstituted in water at a pH of 11. 200 ug of IL-2 was incubated with 25 ml colloidal gold for 24 hours. The colloidal gold bound IL-2 solution was then centrifuged at 14,000 rpm for 20 minutes in a microcentrifuge at room temperature. The supernatant was then removed from the pellet. The IL-2 complex was then placed into each well of 24 well plates and incubated at 37°C for 25 days. Samples were removed from the wells on days 4, 5, 7, 8, 9, 11, 13, 17, 21, 23, and 25. The samples were centrifuged to remove colloidal gold:IL-2 complex. The supernatants were frozen. After the last sample was collected and frozen, all of the samples were batch analyzed in CytImmune Science, Inc.'s CytELISA-2 sandwich EIA for IL.-2. The results of this assay are in Figure 7.

### EXAMPLE X

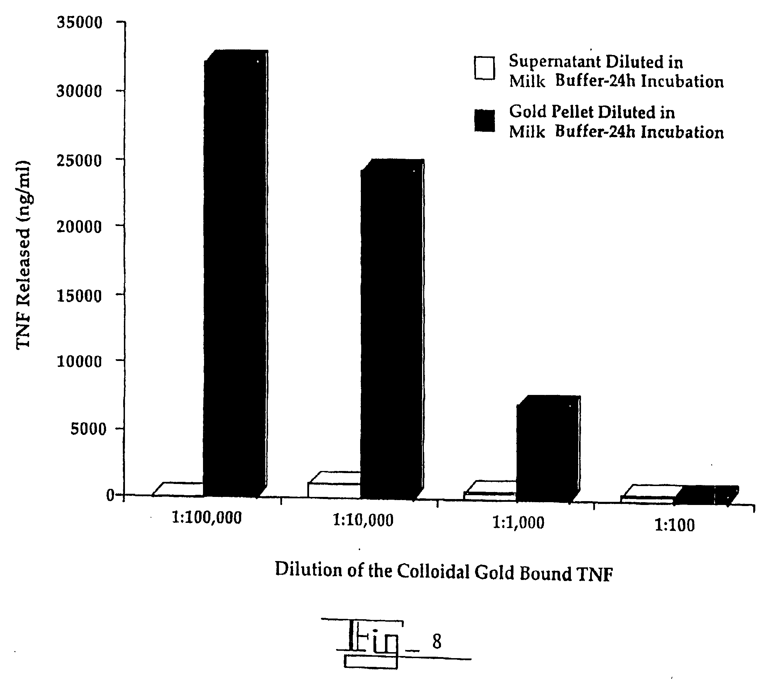

Human TNFα was reconstituted in water at a pH of 11. 200 ug of the TNFα was incubated with the colloidal gold for 24 hours. The TNFα:colloidal gold solution was then centrifuged at 14,000 rpm for 20 minutes in a microcentrifuge at room temperature. The supernatant was then removed from the pellet. The pellet was reconstituted in 1 ml of water and diluted in milk buffer to achieve final concentrations of 1:100, 1: 1,000. 1:10,000, and 1:100,000 and incubated for 24 hours at room temperature. The samples were then subjected to analysis using CytImmune Sciences, Inc.'s CytELISA TNFα assay. The results of this assay are in Figure 8.

This experiment shows that as a result of the dilution, the colloid releases more of the cytokine which is bound to it. Thus, cytokine binding and release by colloidal gold exhibits equilibrium kinetics applicable to *in vivo* situations involving the continuous dilution of the colloidal gold by blood and extracellular fluids.

### EXAMPLE XI

500 ug of recombinant IL-2 was dissolved in 0.5 ml of pH=11 water. The solution was then added to 25 ml of colloidal gold by the method described in Example 9. Balb/C mice were injected (i.p.) with either 0.1 ml of the colloidal gold bound IL-2 or 100 ug of neat IL-2. The mice (4 mice/group/time point) were sacrificed at various time points (0, 0.5, 1.0, 1.5, 2.0, 3.0 and 24 hr) after injection, and trunk blood was collected. The resultant sera was analyzed in CytImmune Sciences. Inc.'s competitive EIA for IL-2. Over the time points tested, the release of IL-2 from the colloidal gold appeared to have a bimodal pattern. (Figure 7) One explanation for this may be the equilibrium of IL-2 released from the colloidal gold into the abdomen chich, subsequently equilibrated with the blood pools.

### EXAMPLE XII

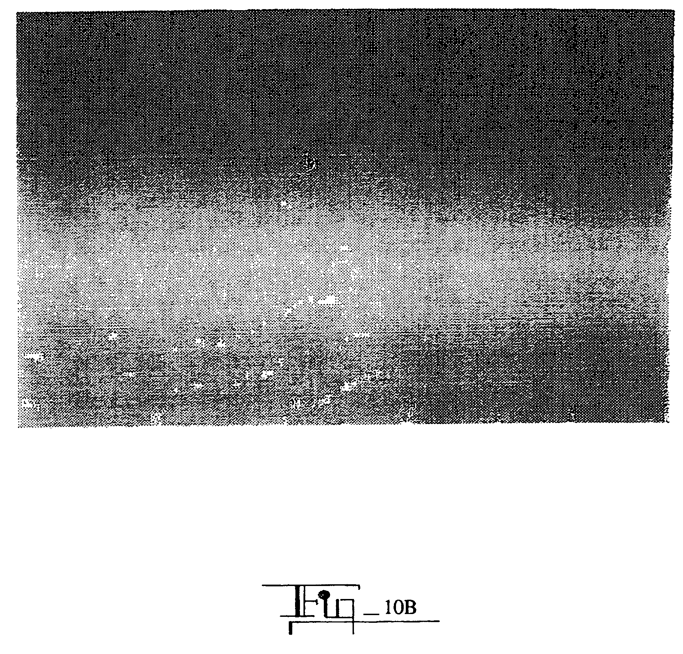

50,000 MCF-7 cells were plated in each well of a 6-well plate in 2 ml of phenol-red-free IMEM WITH 10% CSS. The cells were allowed to grow until they were 70-80% confluent. 200ug of IL-1β was bound to colloidal gold using the method described in Example 9. After binding, the complexed material was centrifuged and blocked with 1% HSA solution. 100 ul of colloidal gold-bound IL-1β complex was added to each well and incubated for 1 to 5 days. The cells were then washed with phenol-red-free IMEM with 10% charcoal-stripped fetal bovine serum (FBS). The binding of the colloidal gold:IL-1β complex to the MCF-7 cells was detected by visualization of the colloidal gold on the cell surface using bright field and phase contrast microscopy. The Colloidal gold/IL-1β remaining on the cell surface was visualized by fluorescence microscopy using rabbit anti-human IL-1β which was internalized into the cells was determined as a negative signal between the fluorescence and bright field images. The results of this assay are illustrated in Figure 10.

Figure 10a is the bright field view of untreated MCF-7 cells (control), and Figure 10b depicts the background fluorescence from the non-specific binding of FITC conjugated antibodies. Figure 10b illustrates the bright field view for MCF-7 cells treated with colloidal gold bound IL-1β, and Figure 10d illustrates FITC fluorescence for MCF-7 cells treated with colloidal gold bound IL-1β complex and internalization of the complex. Some of the colloidal gold:IL-1b complex in these figures is bound to the cell membrane, as indicated by the bright spots, and some has been internalized within the cell, as indicated by the dark spots.

This example shows that one can bind colloidal metal:biologically active factor complex to receptors on the cell surface and that the complex is subsequently internalized within the cell.

### EXAMPLE XIII

50,000 MCF-7 cells were plated in each well of a 6-well plate in 2 ml of phenol-red-free IMEM with 10% CSS. The cells were allowed to grow until they were 70-80% confluent. The cells were then treated with either media, 0.5 ug/ml TNFα, 5.0 ug/ml TNFα, or 50 ug/ml TNFα, 0.5 ug/ml IL-1β, 5.0 ug/ml IL.-1β, or 50 ug/ml IL-1β.

100 ug of IL-1β was bound to colloidal gold by the method described in Example 9. After binding, the complexed material was centrifuged and blocked with 1% HSA solution. 100 ul of colloidal gold bound IL-1β complex was added to each well and incubated for 24-48 hours. The cells were then washed with phenol-red-free IMEM with 10% CSS. The binding of the IL- 1β:colloidal gold:TNFα di-cytokine to the MCF-7 cells was detected by visualization of the colloidal gold on the cell surface using bright field or fluorescence microscopy.

These data indicate that colloidal gold can simultaneously bind two or more biologically-active factors, allowing for the generation of custom complexes which can bind to the cell membrane and provide a targeted drug delivery system.

### EXAMPLE XIV

100 ug each of IL-6, IL-1β and TNFα were simultaneously bound to the same colloidal gold solution using the method described in Example 9. After centrifugation and blocking with HSA solution, the samples were used as unkowns in CytImmune Sciences, Inc.'s sandwich assay for TNF-α. In this assay a monoclonal antibody was used to capture TNF-α in the sample. The monoclonal antibody bound TNF-α was then detected with a rabbit-anti-human TNF-α antibody followed by an enzyme labeled goat-anti-rabbit antibody.

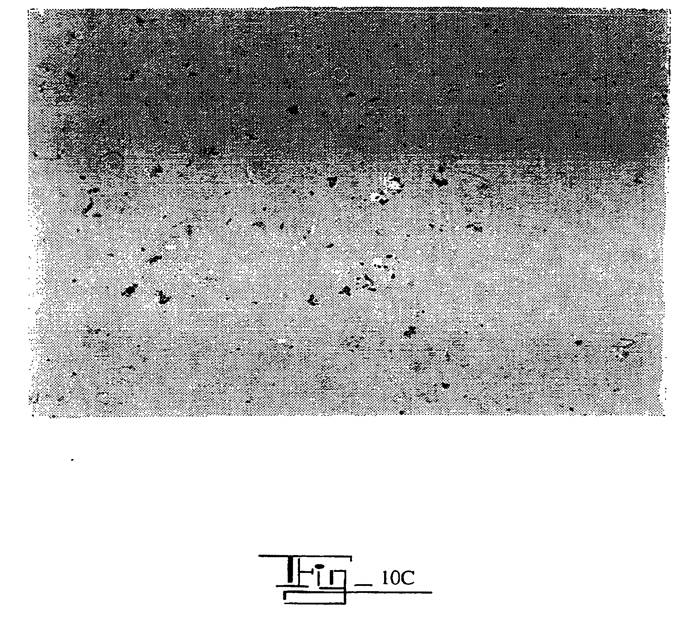

To demonstrate that the same colloidal gold particle bound all IL-6, IL-1β and TNF-α the monoclonal antibody to TNF-α was used to capture 3 sets of quadruplicate samples containing colloidal gold particles which had been simultaneously coated with TNF-α, IL-6 and IL-1β. To demonstrate the tri-cytokine particle, one set of samples was detected with the TNF-α polyclonal antibody, another with the IL-6 polyclonal antibody, and the other with the IL-1β polyclonal antibody. All of the samples were subsequently detected with goat-ant-rabbit antibodies. As, predicted, the colloidal gold bound TNF-α was easily captured and detected with the TNF-α monoclonal/polyclonal antibody binding pairs. In addition, the same samples exhibited a significant amount of immunoreactivity for IL-6 and IL-1β, which was only possible if the three cytokines were bound to the same particles. The results of this assay are illustrated in Figure 11.

These data indicate that colloidal gold can simultaneously bind two or more biologically-active factors, allowing for the generation of custom complexes which can bind to the cell membrane and provide a targeted drug delivery system. These custom complexes can also be used to immunize transgenic mice, generating an immune response to "self antigens" and the production of multiple Mabs. For example, the custom complexes from this experiment could be used to elicit the simultaneous generation of TNF-α, IL-6 and IL-1β Mabs.

### EXAMPLE XV

The following is a general experimental protocol that was followed for binding a molecule to colloidal gold. The molecule was reconstituted in water. 200 µg of the molecule was incubated with 25 mL colloidal gold for 24 hours. The molecule/colloidal gold complex solution was then centrifuged at 14,000 rpm for 20 minutes in a micro centrifuge at room temperature. The supernatant was then removed from the pellet.

### EXAMPLE XVI

50 µg of epidermal growth factor (EGF) was bound to 25 ml of 40nm colloidal gold particles at a pH of 11.0. The solution rocked on rocking platform for 24 hours. 50 µg (added as 50 µl) of targeting cytokine (i.e., IL-1β to target macrophages, IL-2 to target T cells, IL-6 to target B cells, and either TNF alpha or Flt-3 Ligand to target dendritic cells) was added to the EGF/Au solution and rocked for an additional 24 hours. To separate colloidal gold bound and unbound material the solution was then centrifuged at 14,000 rpm. The supernatant was removed and the pellet was reconstituted in 1 ml of water containing 1% human serum albumin.

### EXAMPLE XVII

EGF was bound to colloidal gold (CG) using the procedure in Example 2. Tumor Necrosis Factor-α (TNF-α) was then bound to the EGF/CG complex using the procedure of Example 1 to produce an EGF/CG/TNF-α chimera.

### EXAMPLE XVIII

EGF was bound to colloidal gold (CG) using the procedure in Example 2. Interleukin-6 (IL-6) was then bound to the EGF/CG complex using the procedure of Example 1 to produce an EGF/CG/IL-6 chimera.

### EXAMPLE XIX

EGF was bound to colloidal gold (CG) using the procedure in Example 2. Interleukin-2 (IL-2) was then bound to the EGF/CG complex using the procedure of Example 1 to produce an EGF/CG/IL-2 chimera.

### EXAMPLE XX

The buffy coat was separated from a sample of whole blood as is well known in the art. 100-500 mL of whole blood was collected on heparin. The blood was carefully layered onto a 50% (v/v) ficoll-hypaque solution and centrifuged at 2700 rpm for 7 minutes. The buffy coat, the collection of white blood cells at the serum/ficoll interface, was collected with a Pasteur pipette and placed into 10 mL of PBS containing 0.5 mg/mL heparin. The were centrifuged at 1500 rpm and the pellet washed and recentrifuged. The cells were washed 2X in the PBS solution and centrifuged once again.

The cells were resuspended in RPMI containing either 10% fetal bovine or normal human serum and cultured in 6-well plates at a cell density of 10⁶ cells/well. The cells were then stimulated with 50-100 µL of one or all of the antigen/cytokine mixtures.

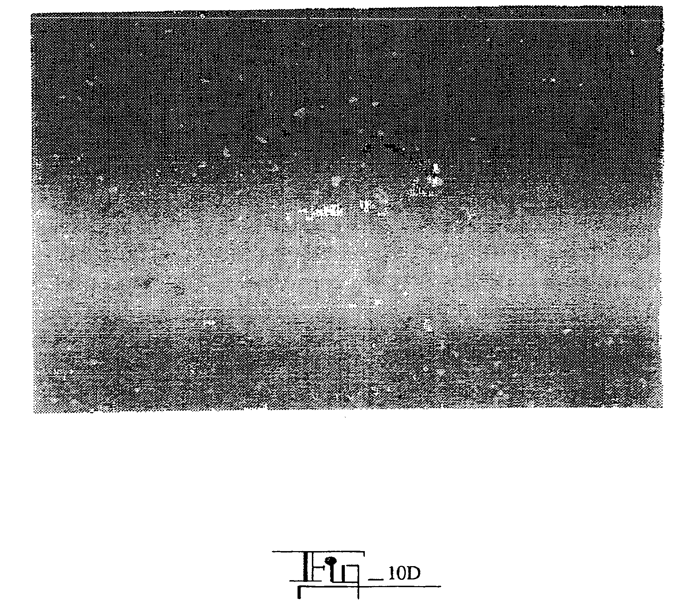

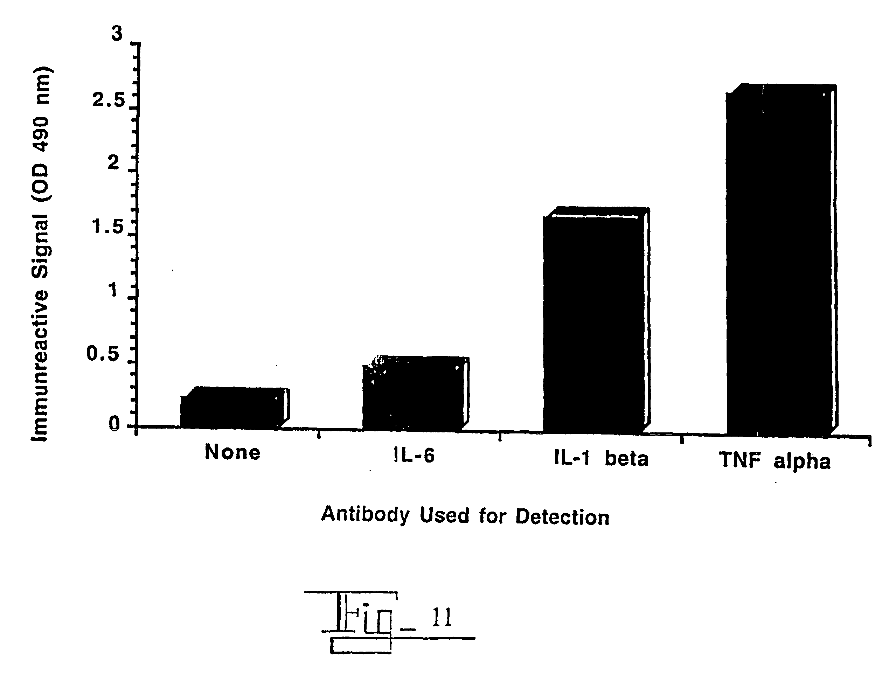

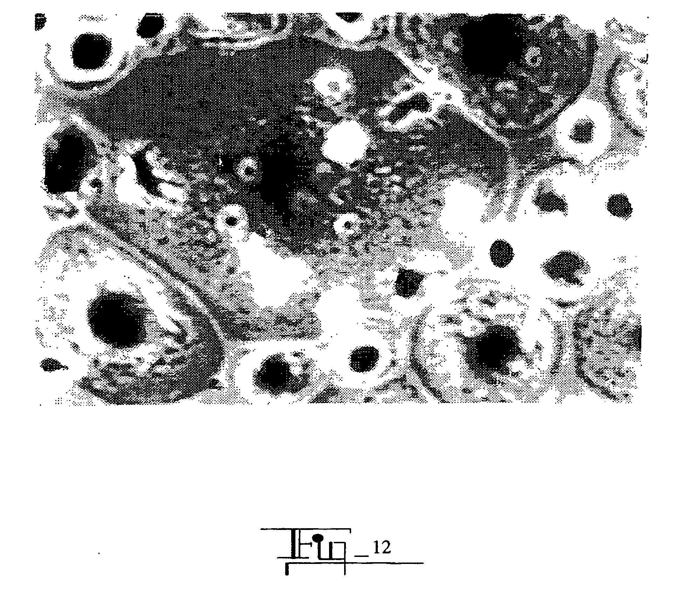

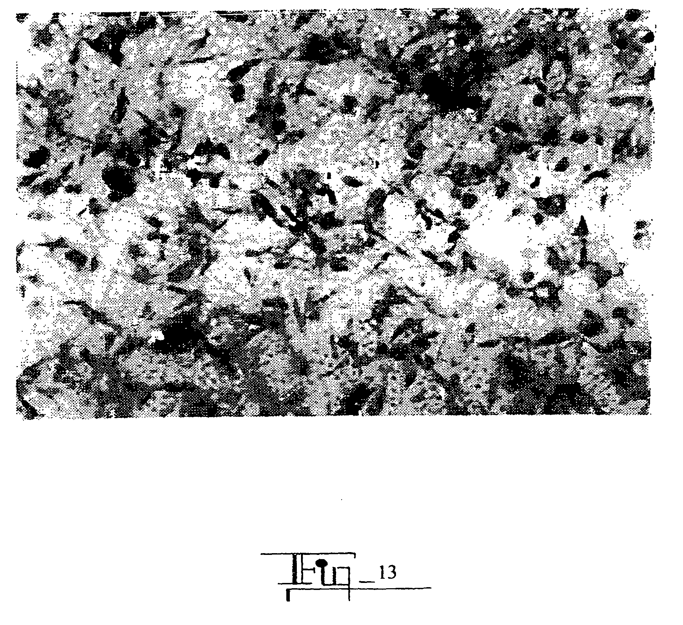

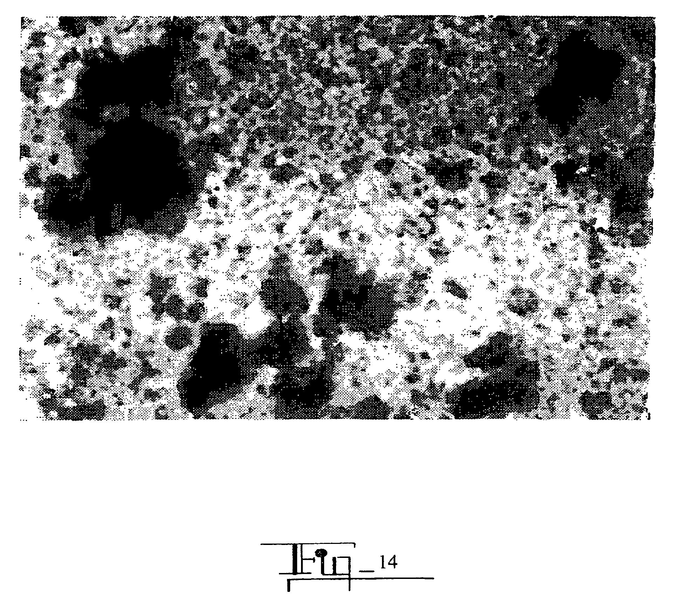

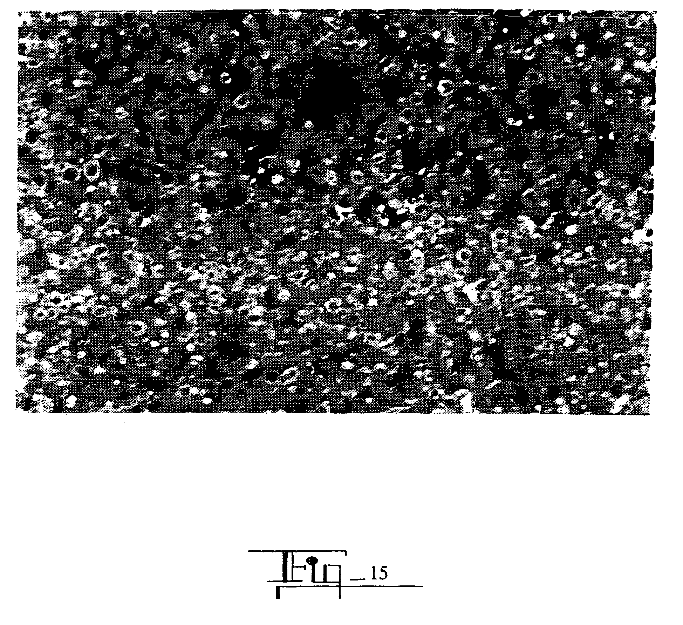

As shown in Figure 12, only macrophages internalized the EGF/CG/IL-1β chimera, while only dendritic cells internalized the EGF/CG/TNF-α chimera (Figure 13). Similarly, only B cells internalized the EGFICG/IL-6 chimera (Figure 14), and only T cells internalized the EGF/CG/IL.-2 chimera (Figure 15).

As shown by this experiment, certain component-specific immunostimulating agents are specific for individual immune components. Thus, it is possible to target specific immune components with component-specific immunostimulating agents, thereby enhancing their immune response resulting in increased activity in the overall immune response.

### EXAMPLE XXI

For this example staphyloccal enterotoxin B was used as the putative antigen/vaccine molecule, since there is evidence that binding the toxin to colloidal gold reduces its toxicity. 500 µg of the toxin was initially bound to 250 ml of 40 nm colloidal gold particles. The colloidal solution was then aliquotted. 50 ug of a targeting cytokine (IL-1β, IL-2, IL-6 and TNFα) was added to one of the aliquots and re-incubated for 24 hours. The toxin-AU-cytokine colloid was centrifuged at 14,000 rpm and the supernatant removed. The pellet was reconstituted to 1 ml of water. The pellet was assayed for cytokine concentration by either sandwich or competitive ELISA. This was done to determine the amount of neat cytokine (unbound) that was to be injected in control animals receiving saline or toxin alone.

The immunization strategy involved simultaneous or sequential administration of neat toxin/cytokine mixture (as composition controls) or the toxin-Au-cytokine chimera. 5 mice/group were injected on days 1. 5 & 9 with either 2.5 ug neat toxin or the same dose of toxin/cytokine mixture bound to colloidal gold. During the 14 day immunization period two additional groups of mice received the neat toxin/cytokine or toxin-Au-cytokine following the schedule provided in Table 1.

**Table 1**

| Day | Group Type | Treatment Injected |
|---|---|---|
| 1 | Control | Neat toxin + Neat IL-1β + Neat TNFα |
| | Gold | Toxin-Au-IL-1β + Toxin-Au-TNFα |
| 5 | Control | Neat toxin + Neat IL-6 |
| | Gold | Toxin-AU-IL-6 |
| 9 | Control | Neat toxin + Neat IL-2 |
| | Gold | Toxin-AU-IL-2 |

All groups were rechallenged with 1 µg of neat toxin alone on day 30. Protective immunization was demonstrated by the reduced or lack of ability of the neat toxin to induce morbidity. The key observation is that the toxin bound to colloidal gold greatly reduced the toxicity of the toxin. Secondly, serum antibody titers to the toxin were 10X higher than those receiving neat treatment alone. However, the serum antibodies of animals receiving the sequential treatment were 100 times greater than the animals receiving the neat treatment. Finally, upon the rechallenge with the neat toxin 100% of the animals treated with toxin died whereas only 20% fatality was observed in the simultaneous group.

Thus the compositions and methods of the present invention can be used to increase the efficacy of a vaccine.

### EXAMPLE XXII

This experiment shows the delivery of the bacterial gene, beta-galactosidase (b-gal), to the human breast cancer cells MCF-7. using colloidal gold which was bound with EGF. For this experiment the β-gal plasmid DNA was directly bound to 40 nm-EGF coupled colloidal gold particles. 25 µ by the methods disclosed in related patent applications. Once the free EGF was separated from the bound fraction, 5 µg of the pSV betagalcatosidase plasmid was incubated overnight with the EGF/Au on a rocking platform. The material was centrifuged and the pellet was reconstituted to 1 ml with water at a pH=11. 100 µl was added to MC F-7 cells which were growing in 6-well culture plates.

The EGF/DNA Au was incubated with the cells for 48 hours to allow for receptor mediated endocytosis of the molecular chimera. Subsequently, the cells were washed 2X with serum free media, 2X with PBS, and then fixed with a 0.25% gluteraldehyde solution (in PBS) for 15 minutes. The fixed cells were washed 4 times with PBS. The beta-galactosidase substrate (X-gal) was added to the cells as a 0.2% solution in a buffer containing 2 mM MgCl₂, 5 mM K₄Fe(CN)₆.3H₂O and 5 mM K₃Fe(CN)₆. The cells and substrate solutions were incubated overnight.

Successful cellular gene transfection and expression is indicated by the conversion of the X-gal substrate to a green colored product. Transfection is confirmed by the presence of a green stain on the cells. In effect, the presence of the stain indicates that the gene was delivered, transcribed and that biologically active enzyme was translated.

It was observed that very few cells stained positive for the β-gal enzyme activity. This may have been due to several factors including poor binding efficiency between the DNA and colloidal gold, lysosomal entrapment and/or poor transcription efficiency.

### EXAMPLE XXIII

This experiment was designed to investigate whether the lack of transfection of Example 2 was due to poor binding of the plasmid DNA to the gold. To test this, gold chimera were generated using EGF as a targeting protein and the DNA binding proteins, such as histones (added as either a heterogeneous mixture of all the isotypes or the individual isotypes), polylysine or protamine sulfate, to adsorb to the DNA. 25 µg of EGF was bound to 40 nm colloidal gold particle as described above. Subsequently histones, polylysine or protamine sulfate were added to the EGF Au solution at various concentrations ranging from 0.1 to 100 µg/ml.

Upon the addition of the DNA binding moiety, we observed the colloidal gold EGF solution became flocculent and subsequently precipitated. The precipitated material was sonicated and centrifuged, and resuspended to a final volume of 1 ml in water. 15 µg of the pSV beta-galactosidase plasmid was incubated overnight or a rocking platform. The material was recentrifuged, the supernatant removed and 0.5 ml of water was added to the pellet. The pellet was re-sonicated and 0.1 ml of the solution was added to either MCF-7 or HS-578-T human breast cancer cells which were grown in 24-well plates using phenol red free IMEM supplemented with 10% charcoal-stripped fetal bovine serum. The cells and gold/protein/DNA chimera were incubated with each other for 48 hours during which it was taken up by the cells.

As can be seen in Figure 16, the beta galactosidase gene was effectively transfected into MCF-7 cells, since the green stain is seen in almost every cell. More interestingly, the HS578-T cells, which are known to possess EGF receptors, only exhibited the extracellular binding of the chimera, see Figure 17. This suggests that the EGF receptor system in this cell line does not undergo internalization.

### EXAMPLE XXIV

Dialysis cassettes were used to show the effects of pH change on colloidal gold particles. Dialysis cassettes (Pierce; Slide-A-Lyzer™; 2000 molecular weight cut-off) were loaded with colloidal gold particles described in the present invention. The cassettes were then placed in one of two MES buffered solutions. MES is 2-[N-morpholinol ethane sulfonic acid]. The pH of the first solution was 7.4 while the pH of the second solution was 5.6 The cassettes were dialyzed for 24 hours. Subsequently, the colloidal gold solutions in the cassettes were collected, placed into 6-well culture plates and photographed. After 24, at pH 7.4, the colloidal gold particles are unchanged and remain suspended in the MES buffer. In cassettes where the pH of the MES buffer was changed to 5.6, after 24, there is black precipitate in the cassettes, indicating the precipitation of the colloidal gold particles. This example described the possible changes in the state of colloidal gold when the pH of the surrounding environment becomes acidic. This is analogous to the acidification of the endosome after internalization.

### EXAMPLE XXV

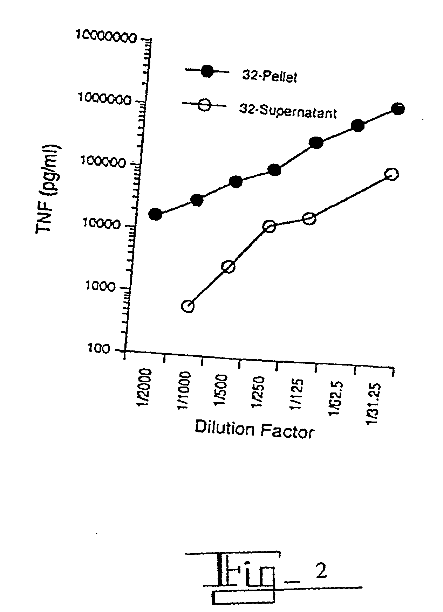

Streptavidin bound to colloidal gold exhibited saturable binding kinetics. For this experiment 500 µg streptavidin was bound to 50 ml of 32 nm colloidal gold for 1 hour. Subsequently, 5 ml of a stabilizing solution (5% PEG 1450,0.1% BSA) was added to the tube and allowed to mix for an additional 30 mm. The sol was centrifuged to remove unbound streptavidin and washed 2 times with 5 ml of the stabilizing solution. After a final spin, the pellet was reconstituted to a volume of 5 ml with the stabilizing solution. 1 ml aliquots were distributed to microfuge tubes. To these tubes increasing amounts of biotinylated human TNF alpha were added. The biotinylated cytokine was incubated with the streptavidin gold for 1 hour. The material was centrifuged at 10,000 rpms for 10 mm. The resultant supernatant was collected and saved for TNF determinations. The pellets from each tube were washed 1 time with stabilizing solution and recentrifuged. The supernatant from this spin was discarded. The pellet was reconstituted to 1 ml with stabilizing solution and both the pellet and initial supernatant were assayed for TNF concentrations using our CYTELISA™ TNF kit. One can see that greater than 90% of the biotinylated TNF immunoreactivity was found in the pellet (Fig.2.) indicating that the biotinylated TNF was captured by the streptavidin bound gold.

### EXAMPLE XXVI

This experiment was to evaluate the feasibility of the streptavidin gold complex as a targeted drug delivery system. In order for this occur the streptavidin conjugated colloidal gold must bind both a biotinylated targeting ligand as well as a biotinylated therapeutic. To investigate this, we performed the following experiment.

100 ml of a 32 nm colloidal gold solution was bound with a saturating concentration of streptavidin. After 1 hour the sol was centrifuged and washed as described above. The colloidal gold bound streptavidin was then bound with sub-saturating concentrations of biotinylated cytokine. The material was vortexed and incubated for 1 hour at room temperature. Afterwards the sol was centrifuged and the pellet incubated with a solution of biotinylated polylysine. After a 1 hour incubation, the sol was re-centrifuged and washed. After a final spin and resuspension (the final volume of the sol was approximately 1 ml) 50 µg of the βgalactosidase reporter gene was incubated with the concentrated streptavidin/biotinylated cytokine/polylysine chimera for I hour. The material was centrifuged to remove unbound plasmid DNA. The final construct (biotin EGF-SAP-Au-biotin polylysine-DNA) was centrifuged at 14,000 rpms. The supernatant was assayed for the presence of DNA by determining its OD at 260 nm. We observed a decrease in the supernatant OD @ 260 nm from 0.95 to 0.25 after the incubation of the plasmid DNA with the biotin EGF-SAP-Au-biotin polylysine construct. The DNA was bound by the biotin EGF-SAP-Au-biotin polylysine-DNA and was centrifuged out of the sol into the pellet. These data show that a new drug delivery system was developed using avidin binding to colloidal gold. Biotinylation of the targeting and delivery payload was then used as the method for binding these molecules to the colloidal gold based drug/gene delivery system.

## Claims

1. A cell delivery system for the targeted delivery of biologically active factors to specific cells, comprising a colloidal metal platform to which at least one biologically active factor and at least one cell-specific targeting molecule are bound, wherein the binding of said at least one biologically active factor and/or the binding of said at least one cell-specific targeting molecule to said colloidal metal platform is mediated by one or more integrating molecules.

2. The delivery system of claim 1, wherein the binding of the at least one biologically active factor and/or the binding of at least one cell-specific targeting molecule to the colloidal metal platform is reversible.

3. The delivery system of claim 1 or 2, wherein the at least one biologically active factor and an integrating molecule form a specific binding pair.

4. The delivery system of claim 1 or 2, wherein the at least one cell-specific targeting molecule and an integrating molecule form a specific binding pair.

5. The delivery system of claim 3 or 4, wherein the specific binding pair is selected from the group consisting of antibody-antigen, receptor-ligand, enzyme-substrate, and streptavidin-biotin.

6. The delivery system of claim 1 or 2, wherein the integrating molecule is a polycationic molecule.

7. The delivery system of claim 6, wherein the polycationic molecule is selected from the group consisting of polylysine, histones, asialoglycoproteins, and protamine sulfate.

8. The delivery system of any of claims 1 to 7, wherein the colloidal metal platform is colloidal gold.

9. The delivery system of any of claims 1 to 8, wherein the at least one biologically active factor is selected from the group consisting of interleukins-1, 2, 3, 4, 5, 6, 7, 8, 10, 11, 12, 13, 15, 16, 17, and 18, lipid A, phospholipase A2, endotoxins, staphylococcal enterotoxin B, type I interferon, type II interferon, tumor necrosis factor-α, transforming growth factor-α, transforming growth factor-β, lymphotoxin, migration inhibition factor, granulocyte macrophage colony-stimulating factor (CSF), monocyte macrophage CSF, granulocyte CSF, vascular epithelial growth factor, basic fibroblast growth factor, angiogenin, heat shock proteins, carbohydrate moieties of blood groups, Rh-factors, fibroblast growth factor, hormones, cell surface receptors, antibodies, nucleic acids, nucleotides, cancer cell specific antigens, autoimmune antigens, mutant p53, tyrosinase, glucose, glycogen, and phospholipids

10. The delivery system of any of claims 1 to 9, wherein the at least one cell-specific targeting molecule is selected from the group consisting of interleukins-1, 2, 3, 4, 5, 6, 7, 8, 10, 11, 12, 13, 15, 16, 17, and 18, lipid A, phospholipase A2, endotoxins, staphylococcal enterotoxin B, type I interferon, type II interferon, tumor necrosis factor-α, transforming growth factor-a, transforming growth factor-β, lymphotoxin, migration inhibition factor, granulocyte macrophage colony-stimulating factor (CSF), monocyte macrophage CSF, granulocyte CSF, vascular epithelial growth factor, basic fibroblast growth factor, angiogenin, heat shock proteins, carbohydrate moieties of blood groups, Rh-factors, fibroblast growth factor, hormones, cell surface receptors, antibodies, nucleic acids, nucleotides, cancer cell specific antigens, autoimmune antigens, mutant p53, tyrosinase, glucose, glycogen, and phospholipids

11. Use of the delivery system of any of claims 1 to 10 for the manufacture of a pharmaceutical composition for treatment of a medical condition selected from the group consisting of bacterial infections, viral infections, cancer, and autoimmune diseases.

## Revendications

1. Système d'administration cellulaire pour l'administration ciblée de facteurs biologiquement actifs dans des cellules spécifiques, comprenant une plate-forme de métal colloïdal à laquelle sont liés au moins un facteur biologiquement actif et au moins une molécule de ciblage spécifique à la cellule,
**caractérisé en ce que**
la liaison du facteur biologiquement actif et/ou la liaison de la molécule de ciblage spécifique à la cellule à la plate-forme de métal colloïdal sont médiées par une ou plusieurs molécules d'intégration.

2. Système d'administration selon la revendication 1,
**caractérisé en ce que**
la liaison du facteur biologiquement actif et/ou la liaison de la molécule de ciblage spécifique à la cellule à la plate-forme de métal colloïdal sont réversibles.

3. Système d'administration selon la revendication 1 ou 2,
**caractérisé en ce que**
le facteur biologiquement actif et une molécule d'intégration forment une paire de liaison spécifique.

4. Système d'administration selon la revendication 1 ou 2,
**caractérisé en ce que**
la molécule de ciblage spécifique à la cellule et une molécule d'intégration forment une paire de liaison spécifique.

5. Système d'administration selon la revendication 3 ou 4,
**caractérisé en ce que**
la paire de liaison spécifique est choisie dans le groupe constitué par une liaison anticorps-antigène, récepteur-ligand, enzyme-substrat et streptavidine-biotine.

6. Système d'administration selon la revendication 1 ou 2,
**caractérisé en ce que**
la molécule d'intégration est une molécule polycationique.

7. Système d'administration selon la revendication 6,
**caractérisé en ce que**
la molécule polycationique est choisie dans le groupe constitué par la polylysine, les histones, les asialoglycoprotéines et le sulfate de protamine.

8. Système d'administration selon l'une quelconque des revendications 1 à 7,
**caractérisé en ce que**
la plate-forme de métal colloïdal est en or colloïdal.

9. Système d'administration selon l'une quelconque des revendications 1 à 8,
**caractérisé en ce que**
le facteur biologiquement actif est choisi dans le groupe constitué par les interleukines 1, 2, 3, 4, 5, 6, 7, 8, 10, 11, 12, 13, 15, 16, 17 et 18, le lipide A, la phospholipase A2, les endotoxines, l'entérotoxine staphylococcique B, l'interféron de type I, l'interféron de type II, le facteur α de la nécrose tumorale, le facteur de croissance transformant α, le facteur de croissance transformant β, la lymphotoxine, le facteur d'inhibition de la migration, le facteur de stimulation des colonies de granulocytes macrophages (CSF), le CSF de monocytes macrophages, le CSF de granulocytes, le facteur de croissance de l'épithélium vasculaire, le facteur de croissance fibroblastique basique, l'angiogénine, les protéines de choc thermique, les fragments carbohydrates des groupes sanguins, les facteurs Rh, le facteur de croissance fibroblastique, les hormones, les récepteurs de surface cellulaire, les anticorps, les acides nucléiques, les nucléotides, les antigènes spécifiques aux cellules cancéreuses, les antigènes auto-immuns, le p53 mutant, la tyrosinase, le glucose, le glycogène et les phospholipides.

10. Système d'administration selon l'une quelconque des revendications 1 à 9,
**caractérisé en ce que**
la molécule de ciblage spécifique à la cellule est choisie dans le groupe constitué par les interleukines 1, 2, 3, 4, 5, 6, 7, 8, 10, 11, 12, 13, 15, 16, 17 et 18, le lipide A, la phospholipase A2, les endotoxines, l'entérotoxine staphylococcique B, l'interféron de type I, l'interféron de type II, le facteur α de la nécrose tumorale, le facteur de croissance transformant α, le facteur de croissance transformant β, la lymphotoxine, le facteur d'inhibition de la migration, le facteur de stimulation des colonies de granulocytes macrophages (CSF), le CSF de monocytes macrophages, le CSF de granulocytes, le facteur de croissance de l'épithélium vasculaire, le facteur de croissance fibroblastique basique, l'angiogénine, les protéines de choc thermique, les fragments carbohydrates des groupes sanguins, les facteurs Rh, le facteur de croissance fibroblastique, les hormones, les récepteurs de surface cellulaire, les anticorps, les acides nucléiques, les nucléotides, les antigènes spécifiques aux cellules cancéreuses, les antigènes auto-immuns, le p53 mutant, la tyrosinase, le glucose, le glycogène et les phospholipides.

11. Utilisation du système d'administration selon l'une quelconque des revendications à 1 à 10, pour la fabrication d'une composition pharmaceutique destinée au traitement d'une pathologie choisie dans le groupe constitué par les infections bactériennes, les infections virales, le cancer et les maladies auto-immunes.

## Patentansprüche

1. Ein Zellabgabesystem für die gezielte Abgabe von biologisch aktiven Faktoren an spezifische Zellen, das eine kolloidale Metallplattform umfasst, an die wenigstens ein biologisch aktiver Faktor und wenigstens ein zellspezifisches Zielmolekül gebunden sind, wobei das Binden des wenigstens einen biologisch aktiven Faktors und/oder das Binden des wenigstens einen zellspezifischen Zielmoleküls an die kolloidale Metallplattform durch ein oder mehrere integrierende Moleküle vermittelt wird.

2. Das Abgabesystem nach Anspruch 1, wobei das Binden des wenigstens einen biologisch aktiven Faktors und/oder das Binden des wenigstens einen zellspezifischen Zielmoleküls an die kolloidale Metallplattform reversibel ist.

3. Das Abgabesystem nach Anspruch 1 oder 2, wobei der wenigstens eine biologisch aktive Faktor und ein integrierendes Molekül ein spezifisches Bindungspaar bilden.

4. Das Abgabesystem nach Anspruch 1 oder 2, wobei das wenigstens eine zellspezifische Zielmolekül und ein integrierendes Molekül ein spezifisches Bindungspaar bilden.

5. Das Abgabesystem nach Anspruch 3 oder 4, wobei das spezifische Bindungspaar aus der Gruppe, die aus Antikörper-Antigen, Rezeptor-Ligand, Enzymsubstrat und Streptavidin-Biotin besteht, ausgewählt wird.

6. Das Abgabesystem nach Anspruch 1 oder 2, wobei das integrierende Molekül ein polykationisches Molekül ist.

7. Das Abgabesystem nach Anspruch 6, wobei das polykationische Molekül aus der Gruppe, die aus Polylysin, Histonen, Asialoglycoproteinen und Protaminsulfat besteht, ausgewählt wird.

8. Das Abgabesystem nach einem der Ansprüche 1 bis 7, wobei die kolloidale Metallplattform kolloidales Gold ist.

9. Das Abgabesystem nach einem der Ansprüche 1 bis 8, wobei der wenigstens eine biologisch aktive Faktor aus der Gruppe, die aus Interleukin-1, -2, -3, -4, -5, -6, -7, -8, -10, -11, -12, -13, -15, -16, -17 und -18, Lipid A, Phospholipase A2, Endotoxinen, Staphylokokken-Enterotoxin B, Typ-1 Interferon, Typ-II Interferon, Tumornekrosefaktor-α, Umwandlungswachstumsfaktor-α, Umwandlungswachstumsfaktor-β, Lymphotoxin, Migrationsinhibierungsfaktor, Granulocyt-Makrophage-Koloniestimulierungsfaktor (CSF), Monocyt-Makrophage CSF, Granulocyt CSF, vaskulärer Epithelialwachstumsfaktor, Fibroblast-Grundwachstumsfaktor, Angiogenin, Wärmeschockproteinen, Kohlenhydrateinheiten von Blutgruppen, Rh-Faktoren, Fibroblastwachstumsfaktoren, Hormonen, Zelloberflächenrezeptoren, Antikörpern, Nukleinsäuren, Nukleotiden, für Krebszellen spezifischen Antigenen, Autoimmun-Antigenen, Mutant p53, Tyrosinase, Glucose, Glycogen und Phospholipiden besteht, ausgewählt wird.

10. Das Abgabesystem nach einem der Ansprüche 1 bis 9, wobei das wenigstens eine zellspezifische Zielmolekül aus der Gruppe, die aus Interleukin-1, -2, -3, -4, -5, -6, -7, -8, -10, -11, -12, -13, -15, -16, -17 und -18, Lipid A, Phospholipase A2, Endotoxinen, Staphylokokken-Enterotoxin B, Typ-I Interferon, Typ-II Interferon, Tumornekrosefaktor-α, Umwandlungswachstumsfaktor-α, Umwandlungswachstumsfaktor-β, Lymphotoxin, Migrationsinhibierungsfaktor, Granulocyt-Makrophage-Koloniestimulierungsfaktor (CSF), Monocyt-Makrophage CSF, Granulocyt CSF, vaskulärer Epithelialwachstumsfaktor, Fibroblast-Grundwachstumsfaktor, Angiogenin, Wärmeschockproteinen, Kohlenhydrateinheiten von Blutgruppen, Rh-Faktoren, Fibroblastwachstumsfaktoren, Hormonen, Zelloberflächenrezeptoren, Antikörpern, Nukleinsäuren, Nukleotiden, für Krebszellen spezifischen Antigenen, Autoimmun-Antigenen, Mutant p53, Tyrosinase, Glucose, Glycogen und Phospholipiden besteht, ausgewählt wird.

11. Verwendung des Abgabesystems nach einem der Ansprüche 1 bis 10 zur Herstellung einer pharmazeutischen Zusammensetzung zur Behandlung einer Krankheit, die aus der Gruppe, die aus bakteriellen Infektionen, viralen Infektionen, Krebs und Autoimmunerkrankungen besteht, ausgewählt wird.
